# EUROPÄISCHE PATENTSCHRIFT

(11) **EP 4 021 372 B1**
(45) Veröffentlichungstag und Bekanntmachung des Hinweises auf die Patenterteilung: **22.11.2023**
(21) Anmeldenummer: 20841668.5
(22) Anmeldetag: 16.12.2020
(51) Int. Cl.: A61J 7/00, G16H 20/10

(54) **EINRICHTUNG UND VERFAHREN ZUR DARREICHUNG EINER POLYMEDIKATION**
DEVICE AND METHOD FOR ADMINISTERING A POLYMEDICATION
DISPOSITIF ET PROCÉDÉ D'ADMINISTRATION D'UNE POLYMÉDICATION

(30) Priorität: 16.12.2019 DE 102019134447
(43) Veröffentlichungstag der Anmeldung: 06.07.2022
(73) Patentinhaber: tantum sana GmbH, 64319 Pfungstadt (DE)
(72) Erfinder: MATSCHKE, Manfred, 68647 Biblis (DE); KALKA, Günther, 64521 Groß-Gerau (DE); MEYER-PHILIPPI, Gerd, 64297 Darmstadt (DE); EYßEN, Wolfgang, 63322 Rödermark (DE); WEYAND, Jens, 54497 Morbach (DE); BRUNNER, Dirk, 81241 München (DE); FRÜHWEIN, Peter, 63322 Rödermark (DE)
(74) Vertreter: Rösler Rasch van der Heide & Partner
(86) Internationale Anmeldenummer: PCT/EP2020/086557
(87) Internationale Veröffentlichungsnummer: WO 2021/122837

(56) Entgegenhaltungen:
- CA-A1- 2 829 547
- KR-A- 20150 039 341
- US-A1- 2010 057 250

## Beschreibung

Die vorliegende Erfindung betrifft eine Einrichtung und ein Verfahren zur Darreichung einer Einzel- oder Polymedikation.

Insbesondere betrifft die vorliegende Erfindung einen Tablettendispenser und ein dazugehöriges Verfahren, die Patienten mit einer Einzel- oder Polymedikation eine pünktliche Einnahme und Dosierung der Medikamente ermöglichen.

Es ist bekannt, dass Medikamente eines Patienten, beispielsweise sein Wochenbedarf, in entsprechende maschinell verpackte Verpackungseinheiten gefüllt werden können. Oftmals werden die in den schlauchförmigen Verpackungseinheiten enthaltenen Medikamente im Anschluss weiter sortiert. Hierbei werden die Schlauchblister von entsprechenden Aufbewahrungs- und Abrollvorrichtungen entnommen, die auf die manuelle Anwendung ausgerichtet sind und in denen die aneinanderhängenden Verpackungseinheiten aufgerollt sind. Auch die nachfolgende Sortierung der einzelnen Verpackungen und Abgabe an die Patienten erfolgt zumeist in der Altenpflege manuell durch das Personal.

In der WO 2016/196 982 A1 ist beispielsweise ein automatisiertes Abgabesystem für Schlauchverpackungen beschrieben, das eine Antriebseinheit umfasst, die eine Abgabe einer Medikamenteneinheit innerhalb eines vorgegebenen Zeitplans ermöglicht.

Aus der US 2014/0 346 184 A1 und der DE 10 2004 025 136 A1 sind weitere herkömmliche Abgabesysteme beschrieben. Auch KR 2015 0039341 A offenbart ein weiteres Abgabesystem.

Der Nachteil der bekannten Systeme ist, dass die Förderung und Ausgabe der Verpackungseinheiten aufgrund der innen liegenden Beförderung der Verpackungseinheiten fehleranfällig ist.

Die Aufgabe der vorliegenden Erfindung ist daher eine Einrichtung und ein entsprechendes Verfahren zur Verfügung zu stellen, welche Einsetz- und Fördertechnik der Verpackungseinheiten verbessert. Ferner soll die hiesige Einrichtung nebst Verfahren dazu dienen, dass die Verwendung durch Personal oder den Patienten selbst verbessert wird, indem die Ausgabe sicherer wird.

Diese Aufgabe wird durch die Merkmale von Anspruch 1 gelöst. Vorteilhafte Weiterbildungen ergeben sich aus den Unteransprüchen.

Die Erfindung bewirkt vorteilhafterweise eine Trennung des Aufbewahrungsbehältnisses von der Vereinzelungseinrichtung. Damit kann ein einfach gestalteter Aufbewahrungsbehälter von geeigneter Stelle, z.B. einer Apotheke oder medizinischem Personal befüllt werden, ohne dass die technisch aufwändige, schwere und teure Vereinzelungseinrichtung dabei ist. Diese kann beim Benutzer verbleiben, der lediglich einen gefüllten Aufbewahrungsbehälter bezieht und diesen mit der Vereinzelungseinrichtung in Wirkverbindung bringt, wobei dabei der Deckel des Aufbewahrungsbehälters geöffnet und eine vorderste Verpackungseinheit entweder manuell oder automatisiert in Eingriff der Vereinzelungseinrichtung gelangt. Von dieser wird der Streifen mit aufeinanderfolgenden Verpackungseinheiten der Trenneinrichtung zugeführt. Entweder automatisch oder auf einen Befehl (z.B. Druck einer Taste) erfolgt dann eine Vereinzelung der vordersten Verpackungseinheit vom Rest, so dass diese vom Benutzer entnommen werden kann.

Wenn der Aufbewahrungsbehälter leer ist, kann dieser einfach von der Vereinzelungseinrichtung abgekoppelt und durch einen neuen, gefüllten Aufbewahrungsbehälter ersetzt werden.

Das Aufbewahrungsbehältnis weist in einer Ausführungsform an einer Ausgabeöffnung eine Auflage mit Zwangsführung für die Aneinanderreihung der Verpackungseinheiten auf. Dies ermöglicht eine gleichmäßige Führung der Verpackungseinheiten.

In einer weiteren Ausführungsform weist das Aufbewahrungsbehältnis eine Kontaktfläche für einen Positionstaster auf, die mit dem an der Vereinzelungseinrichtung angeordneten Positionstaster interagiert, wobei die Position zwischen Aufbewahrungsbehältnis und Vereinzelungseinrichtung bestimmt wird.

Das Aufbewahrungsbehältnis umfasst ferner in einer Ausführungsform eine Anlauframpe, welche beim in Kontakt bringen des Aufbewahrungsbehältnisses mit der Vereinzelungseinrichtung mit einem Hebel des Riemenantriebs der Vereinzelungseinrichtung in Kontakt tritt, wodurch die Auflage des Aufbewahrungsbehältnisses unter ein Förderelement der Vereinzelungseinrichtung geschoben wird.

In einer Ausführungsform der vorliegenden Erfindung weist die Vereinzelungseinrichtung ein Förderelement auf, welches die Aneinanderreihung der Verpackungseinheiten von einer Eingabeöffnung in der Vereinzelungseinrichtung zur Ausgabeöffnung der Vereinzelungseinrichtung transportiert. In einer bevorzugten Ausführungsform umfasst das Förderelement einen federgelagerten Hebel-Riemenantrieb, der mit der Anlauframpe im Aufbewahrungsbehältnis interagiert. In einer besonders bevorzugten Ausführungsform wird die Auflage unter das Förderelement der Vereinzelungseinrichtung geschoben.

Die Vereinzelungseinrichtung kann in einer weiteren Ausführung mindestens ein Lesegerät umfassen, das Informationen der Verpackungseinheiten an einen Rechner überträgt. Bei dem Lesegerät kann es sich vorzugsweise um einen Barcodeleser handeln, der Informationen von der Verpackung scannt und diese an einen Rechner zur Überprüfung und Auswertung übersendet. In einer bevorzugten Ausführungsform umfasst das Lesegerät eine Kamera, welche mit dem Rechner verbunden wird. In einer besonders bevorzugten Ausführungsform wird als Lesegerät die Kamera des Tabletcomputers bzw. Smartphones verwendet.

In einer weiteren Ausführungsform weist die Vereinzelungseinrichtung ferner mindestens einen Sender und mindestens einen Empfänger auf, die die Position der Verpackungseinheiten innerhalb der Vereinzelungseinrichtung bestimmen. Bei den Sendern und Empfängern kann es sich beispielsweise um Lichtschranken handeln. In einer bevorzugten Ausführungsform wird zur Positionserfassung eine Kamera, beispielsweise eine Kamera eines Tabletcomputers bzw. Smartphones verwendet. Um die Positionserfassung durch die Kamera zu erleichtern, kann auf den Verpackungseinheiten ein entsprechendes Muster, beispielsweise optische Referenzpunkte, angebracht sein.

Die Einrichtung umfasst vorzugsweise ein Display, das besonders bevorzugt an der Vereinzelungseinrichtung angeordnet ist und beispielsweise Informationen zum Inhalt der Verpackungseinheiten, Medikation oder dem Patienten darstellen kann. In einer weiteren Ausführungsform ist ein Lautsprecher zur Sprachausgabe vorgesehen.

In einer Ausführungsform der vorliegenden Erfindung weist die Vereinzelungseinrichtung ferner mindestens ein Schneidwerkzeug zur Trennung der einzelnen Verpackungseinheiten auf. In einer bevorzugten Form umfasst die Vereinzelungseinrichtung einen Niederhalter, der die Verpackungseinheiten in einer bestimmten Position hält und eine exakte Trennung der einzelnen Verpackungseinheiten ermöglicht.

In einer Ausführungsform weist der Deckel des Aufbewahrungsbehältnisses eine Verriegelung auf, um eine Manipulation der Verpackungseinheiten zu verhindern, vorzugsweise erfolgt die Verriegelung mittels eines Schlüssels oder ähnlichen Gegenstands, einer Zahlenkombination, biometrischer Identifizierung, eines Smartphones und/oder anderer Identifizierungen.

In einer weiteren Ausführungsform sind ferner bei der Zwangsführung Führungselemente umfasst, die die seitliche Bewegung der Verpackungseinheiten minimieren oder unterbinden, vorzugsweise können diese automatisch oder manuell durch den Benutzer angepasst werden.

Weiterhin umfasst das Element zur Positionserkennung in einer Ausführungsform vorzugsweise mindestens einen Sensor, Taster, Schalter und/oder ein optisches Gerät, wobei es sich bei dem Element zur Positionserkennung um eine Kontaktfläche am Aufbewahrungsbehältnis und einen Positionstaster an der Vereinzelungseinrichtung handelt.

Die vorliegende Erfindung betrifft auch ein Verfahren zur Ausgabe einer bestimmten Verpackungseinheit an einen Patienten mit einer zuvor beschriebenen Einrichtung, wobei das Verfahren folgende Schritte umfasst:
a) In-Kontakt-bringen eines Aufbewahrungsbehältnisses mit einer Vereinzelungseinrichtung, wodurch eine Anlauframpe des Aufbewahrungsbehältnisses einen Hebel-Riementrieb der Vereinzelungseinrichtung nach oben schiebt und eine Auflage des Aufbewahrungsbehältnisses sich unter den Förderriemen schiebt;
b) Öffnen eines Deckels des Aufbewahrungsbehältnisses;
c) Feststellung einer Endposition zwischen Aufbewahrungsbehältnis und Vereinzelungseinrichtung;
d) Absenkung eines federgelagerten Riementriebs auf die Auflage und Aktivierung des Antriebs;
e) Transport der Verpackungseinheit von einer Eingangsöffnung zu einer Ausgangsöffnung innerhalb der Vereinzelungseinrichtung;
f) Bestimmung der Position der Verpackungseinheit innerhalb der Vereinzelungseinrichtung;
g) Fixierung der Verpackungseinheit in einer bestimmten Position; und
h) Trennung einer Verpackungseinheit von der Aneinanderreihung.

Zwischen dem Transport der Verpackungseinheit von einer Eingangsöffnung zu einer Ausgangsöffnung innerhalb der Vereinzelungseinrichtung (Schritt e) und der Bestimmung der Position der Verpackungseinheit innerhalb der Vereinzelungseinrichtung (Schritt f) erfolgt vorzugsweise eine Überprüfung von Daten auf der Verpackungseinheit.

In einer weiteren Ausführungsform der vorliegenden Erfindung überprüft ein Rechner die Funktionen und Positionen der einzelnen Einheiten.

In einer Ausführungsform des hiesigen Verfahrens kann der Deckel des Aufbewahrungsbehältnisses zur Bestückung mit Verpackungseinheiten mittels Schlüssel oder eines ähnlichen Gegenstands, einer Zahlenkombination, biometrischen Identifizierung, eines Smartphones und/oder anderer Identifizierungen geöffnet werden.

Ferner ist umfasst eine Überprüfung von Daten, beispielsweise der Informationen auf und/oder in den Verpackungseinheiten, in einer Ausführungsform einen Abgleich mit internen und /oder externen Datenbanken. In einer bevorzugten Ausführungsform können die Informationen zum Verpackungsinhalt, dem Patienten, den Darreichungsformen und/oder weiteren Informationen auf einem Display der Vereinzelungseinrichtung oder eines externen Geräts wiedergegeben werden.

Die vorliegende Erfindung wird durch die Ausführungsformen in den Ansprüchen gekennzeichnet und durch die Ausführungen in der folgenden Beschreibung und den Zeichnungen näher beschrieben.

### Beschreibung der Zeichnungen

- Fig. 1: Schematische Darstellung einer erfindungsgemäßen Einrichtung mit einem Aufbewahrungsbehältnis und einer Vereinzelungseinrichtung.
- Fig. 2: Schematische Darstellung der Funktionsweise einer bevorzugten erfindungsgemäßen Einrichtung umfassend ein Aufbewahrungsbehältnis und eine Vereinzelungseinrichtung. a) Anfangsposition, wobei ein Aufbewahrungsbehältnis an die Vereinzelungseinrichtung gestellt wird; b) Schwenkposition, wobei beim Einschieben des Aufbewahrungsbehältnisses die Auflage sich unter den Antrieb schiebt, der Deckel beginnt sich zu öffnen; c) Endposition, in der der Deckel für die Förderung geöffnet ist.
- Fig. 3: Schematische Darstellung der Endseite einer Vereinzelungseinrichtung, wobei ein Schneidwerkzeug ausgefahren ist und der Niederhalter sich in seiner aktiven Position befindet.
- Fig. 4: Schematische Darstellung einer bevorzugten Ausgestaltung eines Aufbewahrungsbehältnisses.
- Fig. 5: Schematische Darstellung der automatisierten Öffnung des Deckels des Aufbewahrungsbehältnisses mit einer Schienenführung in Form einer Rampe. a) Erstes in Kontakt treten zwischen dem Zylinder des Aufbewahrungsbehältnisses und der Schienenführung der Vereinzelungseinrichtung; b) Öffnung des Deckels durch hinaufgleiten des am Deckel angeordneten Zylinders auf der als Rampe ausgebildeten Schienenführung der Vereinzelungseinrichtung.

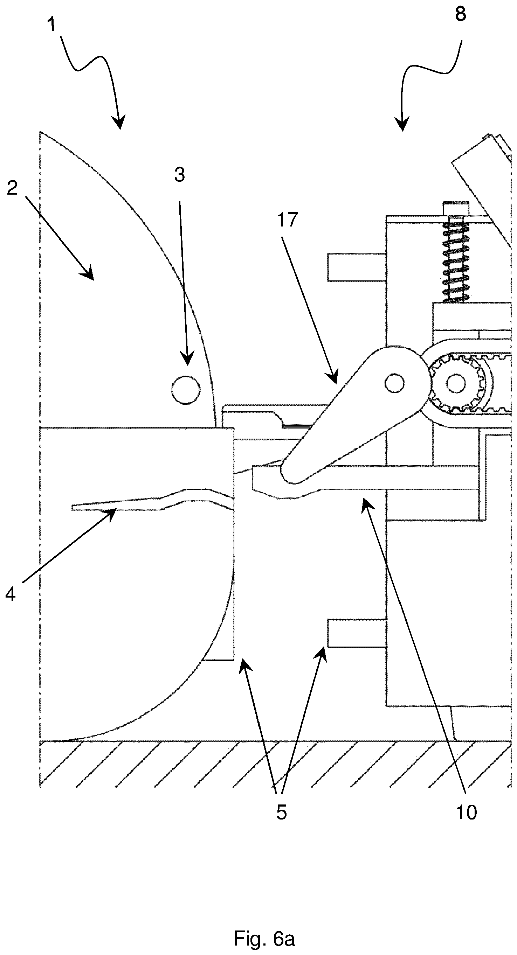

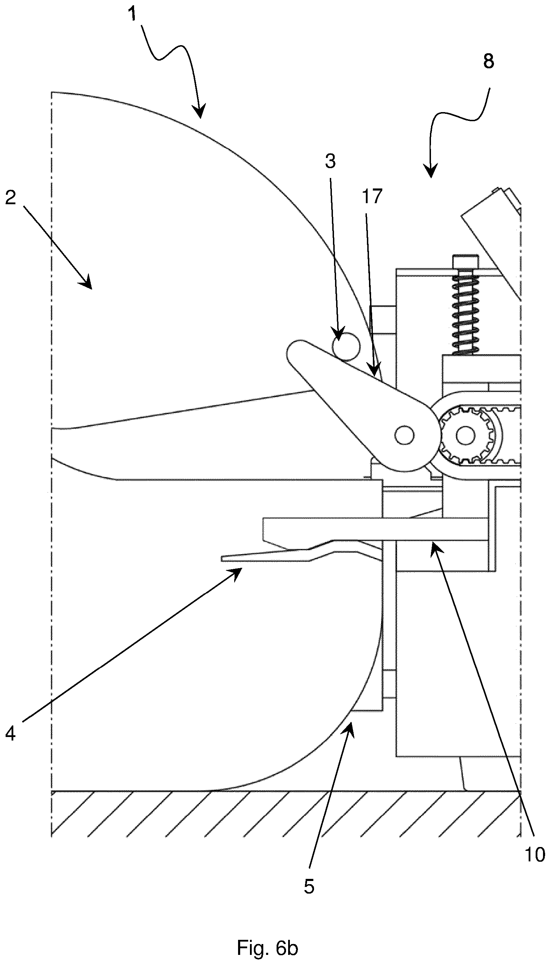

- Fig. 6: Schematische Darstellung eines alternativen Öffnungsmechanismus. a) Heranschieben des Aufbewahrungsbehälters an die Vereinzelungseinrichtung; b) In Kontakt treten eines Kontakts an der Vereinzelungseinrichtung mit dem Deckel des Aufbewahrungsbehältnisses, wodurch ein durch einen Motor betriebenes Element, beispielsweise eine Nocke, bewegt wird und den Deckel öffnet.
- Fig. 7: Perspektivische Darstellung einer zweiten Ausführungsform eines Aufbewahrungsbehältnisses.
- Fig. 8: Detailausschnitt der zweiten Ausführungsform gemäß Fig. 7.
- Fig. 9: Schematische Darstellung eines Einzugsvorgangs für schlauchförmige Verpackungseinheiten mit der Ausführungsform gemäß Fig. 7 und 8.

Die vorliegende Erfindung betrifft eine Einrichtung und ein Verfahren zur Darreichung einer Einzel- oder Polymedikation an eine Person, die ein Patient, Pfleger oder eine andere Person sein kann. Die vorliegende Erfindung ist jedoch nicht beschränkt auf Medikationen im humanen Bereich sondern kann auch die Einzel- oder Polymedikation bei Tieren, wie beispielsweise Hunden oder Pferden umfassen.

Eine Polymedikation kann beispielsweise in Form von unterschiedlichen Tabletten und Rationen in entsprechenden Tablettendosen oder ähnlichen Behältnissen den Patienten dargereicht werden. Ferner kann eine solche Polymedikation auch mittels einzelner Verpackungseinheiten abgegeben werden, welche zumeist vollautomatisiert in diese gefüllt werden. Das sogenannte Verblistern ermöglicht es, dass bestimmte Tabletten sowie Tablettenrationen aus Vorratsbehältern in Verpackungseinheiten eingeschweißt werden. Bei den Verpackungseinheiten handelt es sich zumeist um Einmaltüten, sogenannte Blister, die in einer Endlosreihe aneinanderhängen und eine schlauchförmige Aneinanderreihung der einzelnen Verpackungseinheiten bilden. Eine solche Aneinanderreihung wird zumeist als Streifenblister bezeichnet. Streifenblister können beispielsweise auch eine äquidistante Anordnung der Medikamente aufweisen. Die Aneinanderreihung der Verpackungseinheiten bzw. die Streifenblister werden üblicherweise an die Apotheken geliefert, wo diese dann aufgerollt und üblicherweise in Transportboxen, sogenannten Dispensern, aufbewahrt werden sowie zur manuellen Abgabe aus diesen bereitgestellt werden.

An den einzelnen Verpackungseinheiten, die die Medikamente enthalten, sind üblicherweise die wesentlichen Medikationsdaten sowie weitere wichtige Daten zur Sicherheit und Kontrolle, beispielsweise auch personenbezogene Daten, aufgebracht, um eine korrekte Verabreichung der patientenindividuellen Medikation zu gewährleisten. Bei den Medikamenten, die dargereicht werden, kann es sich um Medikamente in Form von Tabletten, Dragees, Kapseln oder dergleichen handeln.

Es ist insbesondere die Aufgabe der vorliegenden Erfindung, eine Einrichtung zur Verfügung zu stellen, die die patientenindividuell zusammengestellten Verpackungseinheiten auch patientenindividuell bereitstellt, ohne eine Manipulation durch den Patienten zu erlauben. Die Einrichtung soll insbesondere für Patienten von Nutzen sein, die regelmäßig Medikamente einnehmen oder an klinischen Studien teilnehmen. Für eine praktische Nutzbarkeit für ältere Menschen zur selbstständigen Verwendung im häuslichen Umfeld ist es besonders wichtig, dass ein Darreichungsprozess der Medikation einfach, zuverlässig und sicher ist.

Hierbei werden die handelsüblichen Schlauchblister, die in den Blisterzentren patientenindividuell bestückt werden und anschließend an Apotheken oder entsprechende Einrichtungen, wie Krankenhäuser, Rehabilitationszentren oder Seniorenheime geliefert werden, in ein Aufbewahrungsbehältnis 1 eingebracht. Die Verpackungseinheiten weisen dabei eine chronologische Reihenfolge auf, die nach dem Zeitpunkt der Verabreichung sortiert sind. Die Begriffe "Verpackungseinheiten", "Schlauchblister", "Streifenblister" und "Blisterrolle" werden im Folgenden austauschbar verwendet und beschreiben die Aneinanderreihung der Einmaltüten durch beispielsweise Verschweißungen, in denen sich Medikamente befinden, wobei die einzelnen Einheiten durch Schneiden, Reißen oder ähnliche Mechanismen voneinander getrennt werden müssen. Auch die Begriffe "Verpackungseinheit" und "Blister" werden austauschbar verwendet und beschreiben eine einzelne Einmaltüte, in welcher sich mindestens ein Medikament, vorzugsweise mehrere Medikamente befinden. Die Verpackungseinheiten können grundsätzlich aus jedem beliebigen geeigneten Material hergestellt werden und eine beliebige Größe aufweisen, jedoch sind vorzugsweise Materialien für die Einrichtung vorgesehen, die Feuchtigkeitsresistent sind, um die Medikamente zu schützen. Ferner weisen die aneinander angeordneten Verpackungseinheiten eine Breite von 1 cm bis 10 cm, vorzugsweise zwischen 2 cm bis 8 cm, besonders bevorzugt zwischen 6 cm und 8 cm auf.

In einem bevorzugten Verfahren werden die Verpackungseinheiten durch Apotheken oder entsprechende Einrichtungen, wie Krankenhäuser, Rehabilitationszentren oder Seniorenheime in ein Aufbewahrungsbehältnis 1 eingeführt. Natürlich kann eine solche Einführung der Verpackungseinheiten in das Aufbewahrungsbehältnis 1 jedoch auch durch den Patienten selbst, einen Angehörigen oder eine dritte Person erfolgen, wobei darauf zu achten ist, dass eine Fehlmedikation vermieden wird.

Bei dem Aufbewahrungsbehältnis handelt es sich um einen Dispenser, d.h. einen Dosierspender, eine Patrone oder ein Magazin, der die Aufbewahrung von Verpackungseinheiten ermöglicht. Das Aufbewahrungsbehältnis 1 ist mit einem Deckel 2 verschlossen und umfasst mindestens eine Öffnung an einer Seitenfläche des Gehäuses zur Ausgabe eines freien Endes der Verpackungseinheiten.

Der bewegliche Deckel 2, verschließt eine Öffnung zum Inneren des Aufbewahrungsbehältnisses 1, durch welche die Verpackungseinheiten in das Gehäuse des Aufbewahrungsbehältnisses 1 eingebracht werden können und bei einer Interaktion mit einer Fördereinrichtung 9 das freie Ende der Verpackungseinheiten zusätzlich freigibt. Der Begriff "Deckel" umfasst hierbei jede erdenkliche Form einer Abdeckung. Der Deckel kann hierbei auch einen Teil des Aufbewahrungsbehältnisses 1 darstellen, wobei nur eine kleine Öffnung freigelegt wird, durch welche die Verpackungseinheiten in dieses eingebracht werden. Der Deckel kann auch aus unterschiedlichen Materialen bestehen. Der Deckel 2 befindet sich vorzugsweise auf der Oberseite des Gehäuses, wobei eine Verbindung zwischen dem Deckel 2, der die Oberseite des Gehäuses darstellt und dem Gehäuse selbst, vorzugsweise an der, der Ausgabeöffnung gegenüberliegenden Gehäuseseite, angeordnet ist. Bei der Verbindung kann es sich um jede bekannte bewegliche Verbindung handeln, die eine Öffnung des Deckels 2 vom Gehäuse ermöglicht, wie z.B. ein um eine Achse drehbares Gelenk. Der Deckel 2 des Aufbewahrungsbehältnisses ist in einer Ausführungsform verriegelt, um eine Manipulation der Verpackungseinheiten zu verhindern. Eine entsprechende Verriegelung kann durch ein Schlüssel-Schloss-Prinzip gesichert sein, wobei eine Entriegelung neben einem Schlüssel oder ähnlichen Gegenstand auch durch eine Zahlenkombination, biometrische Identifizierungen oder andere Identifizierungen erfolgen kann.

Innerhalb des Aufbewahrungsbehältnisses 1 kann ein Halteelement angeordnet sein, das eine Achse bildet, über welche die Verpackungseinheiten aufgerollt sind, wodurch die Verpackungseinheiten in einer bestimmten Position gehalten werden und abgerollt werden können. Das Halteelement ist hierbei parallel zum Boden des Gehäuses des Aufbewahrungsbehältnisses 1 und relativ mittig innerhalb von diesem angeordnet, um ein leichtes Abrollen der Verpackungseinheiten von der Rolle zu ermöglichen. Bei dem Halteelement handelt es sich vorzugsweise um eine zylinderförmige Achse, die lösbar zwischen den Seitenwänden des Gehäuses angeordnet ist, um einen Austausch der Verpackungseinheiten zu ermöglichen.

Das Gehäuse des Aufbewahrungsbehältnisses 1 kann grundsätzlich jede erdenkliche Form aufweisen. Aufgrund der Torus-förmigen Aufrollung der Verpackungseinheiten weist das Aufbewahrungsbehältnis 1 jedoch vorzugsweise eine rundliche Form auf, wobei der Boden des Gehäuses zur besseren Standfestigkeit und die hintere Gehäuseseite abgeflacht sind.

Das Aufbewahrungsbehältnis 1 wirkt mit einer Vereinzelungseinrichtung 8 zusammen und bildet zusammen mit dieser eine Einrichtung, die eine Abgabe einer Verpackungseinheit aus einer Vielzahl schlauchförmig aneinandergereihter Verpackungseinheiten, ermöglicht.

Das Zusammenwirken der beiden Elemente wird durch ein In-Kontakt-bringen des Aufbewahrungsbehältnisses 1 mit der Vereinzelungseinrichtung 8 erreicht. Ein solcher Kontakt kann beispielsweise direkt durch das Zusammenschieben der beiden Elemente zueinander bewirkt werden, wobei die Seite mit der Auflage 7 am Aufbewahrungsbehältnis 1 zu einer Seite mit einer Eintrittsöffnung an der Vereinzelungseinrichtung 8 zeigt. Der Kontakt kann jedoch auch indirekt sein, indem ein weiteres Element zwischengeschaltet ist und beispielsweise die Zusammenführung beider Elemente an eines der beiden Elemente oder beide Elemente weitergibt.

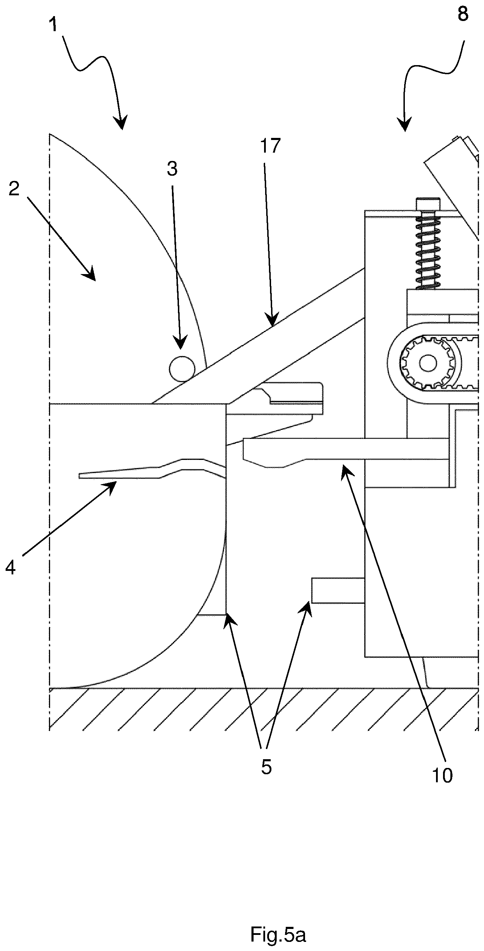

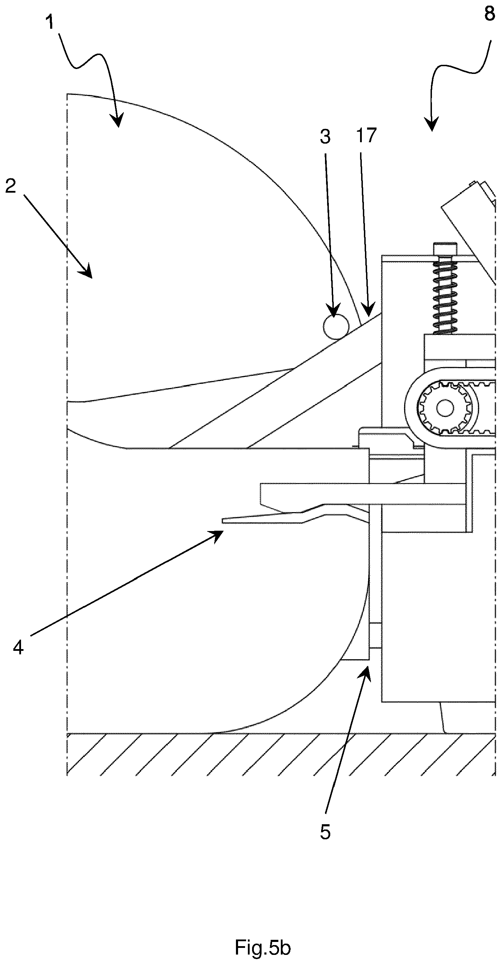

Durch das Zusammenschieben des Aufbewahrungsbehältnisses 1 mit der Vereinzelungseinrichtung 8 wird ein Hebel 10 der Vereinzelungseinrichtung durch eine Anlauframpe 4 des Aufbewahrungsbehältnisses nach oben gedrückt. Dies hat zur Folge, dass ein am Deckel 2 angeordneter Zylinder 3 die Öffnung des Deckels 2 des Aufbewahrungsbehältnisses veranlasst. Eine solche Öffnung erfolgt vorzugsweise automatisiert. Eine solche automatisierte Öffnung erfolgt hierbei durch einen Motor, der beispielsweise ein Schrittmotor, Servomotor, Elektromotor oder Getriebemotor sein kann, der mit einem Antrieb, wie beispielsweise Spindelantrieb oder Klappantrieb zusammenwirkt. Jedoch kann auch eine einfache Schienenführung vorgesehen sein, wie beispielsweise der Fig. 5 zu entnehmen ist.

Der Deckelautomatismus kann ferner zusätzlich Elemente zur Positionserkennung umfassen, die die Position des Deckels 2, bzw. die maximale Öffnung bestimmen. Hierfür können neben entsprechenden Potentiometern, Mikrocontroller, Mikroschalter sowie auch Recheneinheiten verwendet werden. Bei dem Deckelautomatismus ist auch eine Erkennung mittels einer Kamera denkbar. Dies kann durch geschickte Strahlführung, beispielsweise mithilfe eines Spiegels und/oder einer Kamera eines Tabletcomputers oder Smartphones erfolgen. Bei Verwendung eines solchen ist es nicht notwendig, dass das gesamte Aufbewahrungsbehältnis 1 nebst Deckel 2 aufgenommen wird. Vorzugsweise ist die Erkennung eines kleinen Bildausschnitts für die Erkennung der Deckelposition ausreichend.

Der Deckel 2 dient dem Schutz der Verpackungseinheiten vor äußeren Einflüssen und zusätzlich dem Schutz der Manipulation dieser.

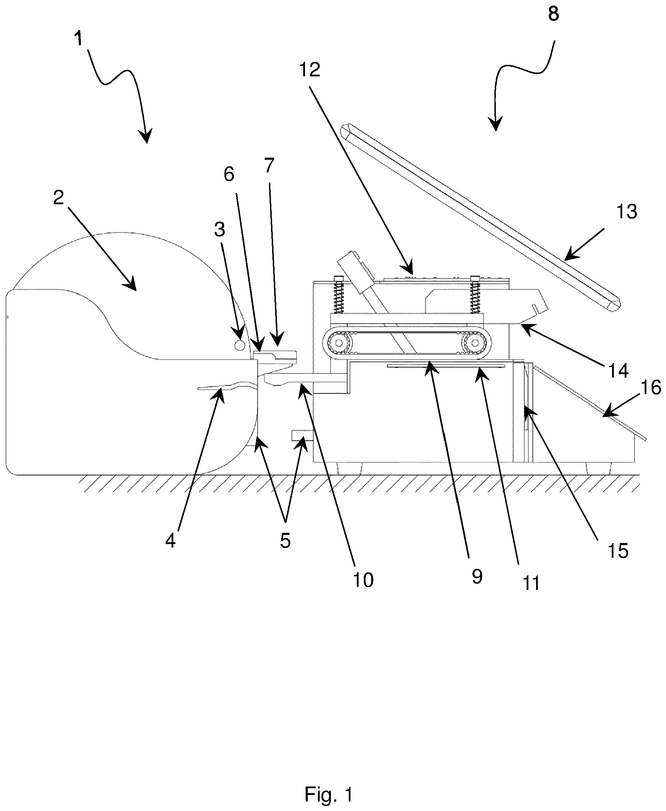

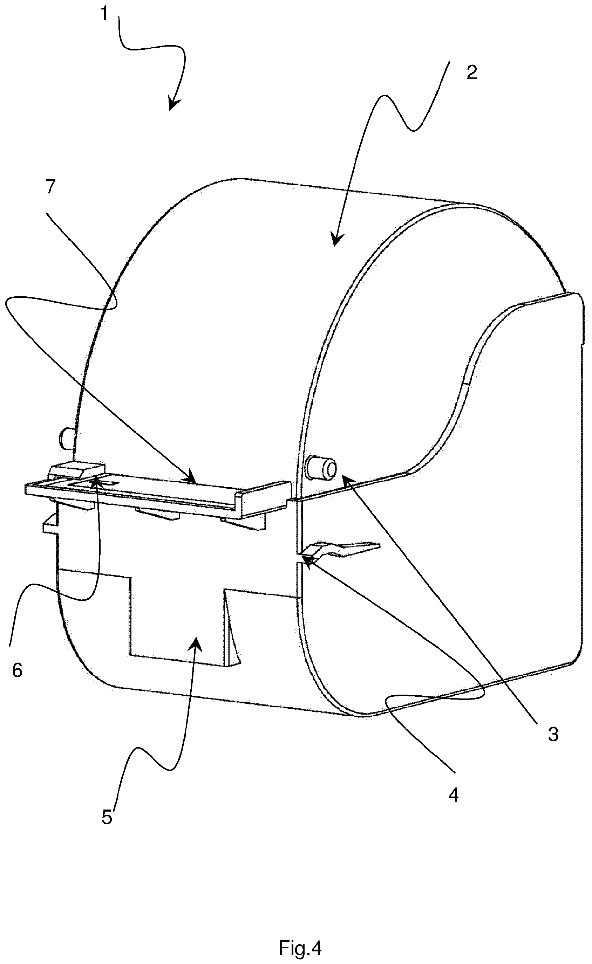

Das Aufbewahrungsbehältnis 1 weist wie zuvor erwähnt im Gehäuse eine Ausgabeöffnung auf, welche auf einer Seite des Gehäuses angeordnet ist. Wie der Fig. 1 und 4 zu entnehmen ist, befindet sich diese an der Gehäuseseite, die der Vereinzelungseinrichtung 8 zugewandt ist, vorzugsweise an der Gehäuseseite, die der abgeflachten Seite gegenüberliegt. An der Ausgabeöffnung ist vorzugsweise eine Auflage 7 angeordnet, über welche die Verpackungen aus dem Aufbewahrungsbehältnis 1 geführt werden. Dementsprechend ist die Ausgabeöffnung des Aufbewahrungsbehältnisses und die Auflage 7 zentralisiert an dem Aufbewahrungsbehältnis 1 angeordnet, wie beispielsweise der Fig. 1 und 4 entnommen werden kann. In einer bevorzugten Ausführungsform ist die Öffnung mit der Auflage 7 an der oberen Seite des Gehäuses angeordnet, die durch den Deckel 2 verschlossen wird, wodurch diese in unmittelbarem Kontakt zueinander stehen können.

Die Auflage 7 für die Verpackungseinheiten kann ferner eine Zwangsführung 6 umfassen. Diese kann Führungselemente umfassen, die seitliche Bewegungen der Verpackungseinheiten minimieren oder unterbinden. Bei dieser Führung kann es sich um jedes Element handeln, das die Seiten der Auflage 7 verschließt, wie beispielsweise Schienen, Kanten sowie ähnliche Elemente oder Hervorhebungen, die die ansonsten flache Auflage 7 an den Seitenrändern abschießen. Die Zwangsführung 6 ist hierbei vorzugsweise derart ausgebildet, dass sie automatisch oder manuell durch den Benutzer angepasst werden kann. Die Auflage 7 mit Zwangsführung 6 erlaubt eine gleichmäßige Führung und somit Entnahme der Verpackungseinheiten aus dem Aufbewahrungsbehältnis 1.

Die Vereinzelungseinrichtung 8, die mit dem Aufbewahrungsbehältnis 1 zusammenwirkt, hat mehrere Aufgaben. Sie dient der Überprüfung der Patientendaten sowie dem Abgleich der verwendeten Verpackungseinheit und Trennung einzelner bzw. mehrerer zusammenhängender Verpackungen.

Weiterhin kann das Aufbewahrungsbehältnis 1 an der Vorderseite, d. h. der Seite, die die Ausgabeöffnung aufweist und der Vereinzelungseinrichtung 8 zugewandt ist, oder an der Vorderseite der Vereinzelungseinrichtung 8, d. h. der Seite, an der die Verpackungseinheiten aus dem Aufbewahrungsbehältnis 1 in die Vereinzelungseinrichtung 8 geführt werden, ein Element zur Positionserkennung 5 zwischen dem Aufbewahrungsbehältnis 1 und der Vereinzelungseinrichtung 8 aufweisen. Das Element zur Positionserkennung 5 kann jedes Element sein, das den Abstand bzw. die Entfernung zwischen dem Aufbewahrungsbehältnis 1 und der Vereinzelungseinrichtung 8 bestimmen kann oder den Kontakt zwischen beiden erfasst. Dieses Element 5 kann ein mechanisches oder elektronisches Bauteil sein, das beispielsweise durch Druck ausgelöst wird. Dies kann beispielsweise ein Sensor, Taster und/oder Schalter sein. Auch ist eine optische Erkennung möglich.

Wie den Fig. 1 und 5 zu entnehmen ist, handelt es sich bei dem Element zur Positionserkennung 5 vorzugsweise um eine Kontaktfläche am Aufbewahrungsbehältnis 1 und einen Positionstaster an der Vereinzelungseinrichtung 8, der bei einem bestimmten Druck, d. h. bei Kontakt zwischen dem Aufbewahrungsbehältnis 1 und der Vereinzelungseinrichtung 8 die Endposition der beiden Elemente zueinander erkennt. Diese Endposition signalisiert vorzugsweise dem Deckel 2, dass dieser sich nicht weiter öffnen soll. Ferner wird ein Signal an ein Förderelement gesendet, sodass dieses aktiviert wird und die Verpackungseinheit aus dem Aufbewahrungsbehältnis 1 in die Vereinzelungseinrichtung 8 einzieht. Sobald Sensoren erkennen, dass eine gewünschte Position der Verpackungseinheit innerhalb der Vereinzelungseinrichtung erreicht wurde, so wird das Förderband automatisch angehalten. Das Element zur Positionserkennung 5 kann jedoch eine beliebige Ausführung aufweisen, beispielsweise kann auch lediglich ein Positionstaster an dem Aufbewahrungsbehältnis 1 oder der Vereinzelungseinrichtung 8 angeordnet sein, der direkt mit dem jeweils anderen Element interagiert, welches den Positionstaster nicht umfasst.

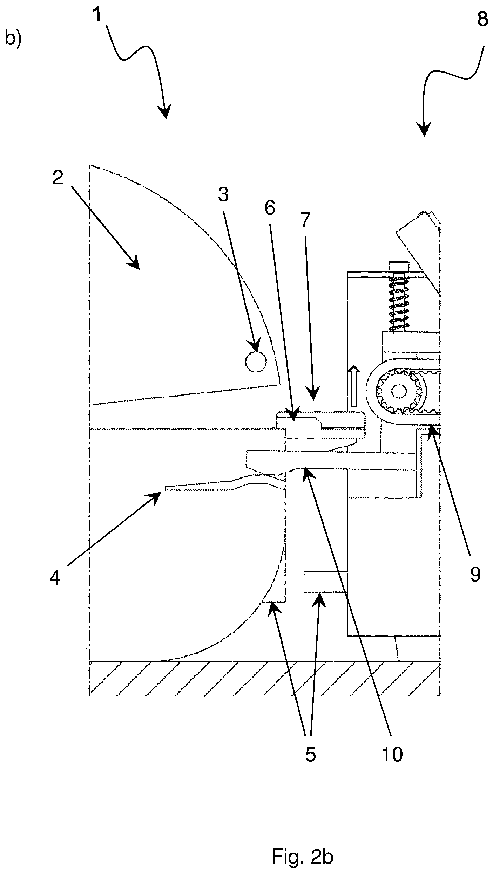

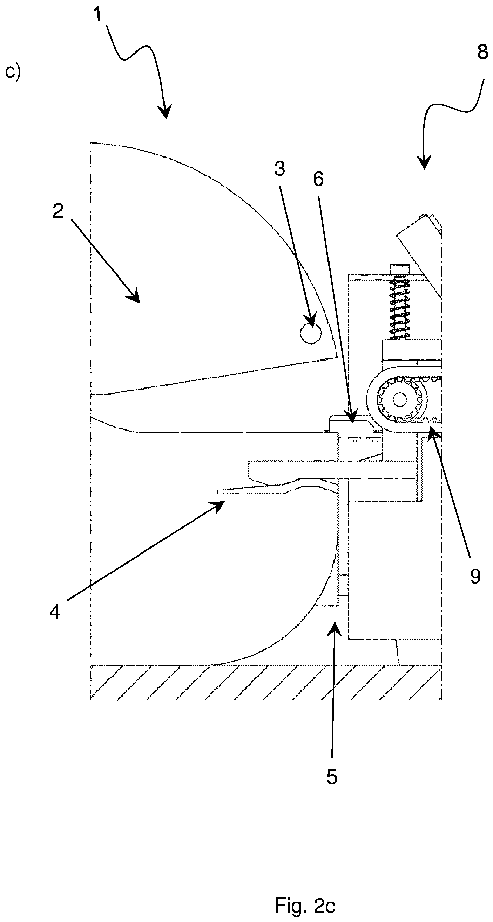

Das Aufbewahrungsbehältnis 1 umfasst in einer weiteren Ausführungsform mindestens eine Rampe 4, welche beim In-Kontakt-bringen des Aufbewahrungsbehältnisses 1 mit der Vereinzelungseinrichtung 8 mit einem Hebel 10 der Vereinzelungseinrichtung in Kontakt tritt. Diese Anlauframpe 4 ist auf der Vorderseite unterhalb der Öffnung und unterhalb der Auflage 7 für die Verpackungseinheiten im Seitenbereich des Gehäuses angeordnet. In einer bevorzugten Ausführungsform befindet sich an beiden Seitenbereichen jeweils eine Anlauframpe 4. Die Anlauframpe 4 ist vorzugsweise derart ausgebildet, dass sie eine leichte Wellenform bildet, welche mit einem Hebel 10, der eine komplementäre Wellenform aufweist, interagieren kann. Beim Zusammenführen des Aufbewahrungsbehältnisses 1 und der Vereinzelungseinrichtung 8 wird der Hebel 10 der Vereinzelungseinrichtung 8 durch die Anlauframpe 4 nach oben gehoben, wodurch die Auflage 7 des Aufbewahrungsbehältnisses unter die Fördereinrichtung 9 der Vereinzelungseinrichtung 8 geschoben wird. Hierdurch werden die Verpackungseinheiten in die Vereinzelungseinrichtung 8 gezogen. Bei der Fördereinrichtung 9 kann es sich um jeden bekannten Fördermechanismus handeln, der es ermöglicht, die Verpackungseinheiten innerhalb der Vereinzelungseinrichtung 8 von einer Eingangsöffnung zu einer Verpackungseinheitausgabe zu transportieren. Hierbei kann es sich beispielsweise um eine oder mehrere Walzen handeln, die vorzugsweise aus Schaumstoff gefertigt sind und/oder gummiert sind. Bei der Verwendung von schaumstoffummantelten Walzen kann die Kontur der Verpackungseinheiten ausgeglichen werden, d.h. solche Walzen können entlang der gesamten Breitenerstreckung der Verpackungseinheiten verlaufen und eine gleichmäßige Förderung der Verpackungseinheiten bewirken. In einer bevorzugten Ausführungsform, wie sie beispielsweise den Fig. 1 und 5 zu entnehmen ist, handelt es sich bei der Fördereinrichtung 9 um einen federgelagerten Riemenantrieb. Eine solche Federlagerung der Fördereinrichtung 9 ermöglicht es, dass beim In-Kontakt-treten des Aufbewahrungsbehältnisses 1 und der Vereinzelungseinrichtung 8, in der Schwenkposition, wie sie beispielsweise der Fig. 2b zu entnehmen ist, das Förderelement durch den Hebel 10 leicht zur Oberseite der Vereinzelungseinrichtung 8 angehoben wird. Durch die Anhebung des Förderelementes wird die Auflage 7 unter dieses befördert, wodurch die Verpackungseinheiten von einer Eingabeöffnung in der Vereinzelungseinrichtung 8 zur Verpackungseinheitausgabe 16der Vereinzelungseinrichtung 8 transportiert werden. Dementsprechend interagiert der Hebel-Riemenantrieb 10 der Vereinzelungseinrichtung 8 mit der Anlauframpe 4 im Aufbewahrungsbehältnis. Erreicht die Positionierung der beiden Elemente, nämlich des Aufbewahrungsbehältnisses 1 und der Vereinzelungseinrichtung 8, zueinander ihre Endposition, wie beispielsweise in Fig. 2c dargestellt, so wird in einer Ausführungsform ein Signal an das Förderelement geleitet, das diesem eine Absenkung signalisiert. Die Blister können Tabletten unterschiedlicher Anzahl, Form und Größe aufweisen. Um Schädigungen der Tabletten durch einen zu hohen Anpressdruck der Rampe mit dem Förderelement zu verhindern, ist in einer Ausführungsform ein Sensor vorgesehen, der einen Widerstand durch die Tabletten erkennt und ein weiteres Absenken des Antriebs und somit einen zu hohen Anpressdruck verhindert. Ferner ist in einer weiteren Ausführungsform vorgesehen, dass die Federlagerung der Fördereinrichtung 9 diesen auch in einem bestimmten Abstand zu den Blistern und den darin enthaltenen Tabletten hält, sodass die Verpackungen transportiert werden, es jedoch nicht zur Schädigung der Tabletten und/oder Störung des Transportmechanismus kommt.

An der Ausgabeöffnung 16 der Vereinzelungseinrichtung 8 werden eine Verpackungseinheit oder mehrere zusammenhängende Verpackungseinheiten für einen Patienten für einen bestimmten Tag, einen Zeitraum oder bestimmte Uhrzeiten ausgegeben.

Innerhalb der Vereinzelungseinrichtung 8 ist in einer Ausführungsform ein Lesegerät umfasst, das Informationen, welche beispielsweise auf den Verpackungseinheiten angeordnet sind, an einen Rechner überträgt.

Entsprechende Informationen, die auf der Verpackungseinheit angeordnet sein können, umfassen insbesondere den Namen des Patienten, Adresse des Patienten, Datum der Verpackung, Datum der Herstellung, Verfallsdatum des bzw. der Medikamente, Datum der Verabreichung, Uhrzeit der Verabreichung, Anweisungen zur Einnahme des Medikaments, Warnhinweise, Verpackungsinhalt, Dosierung der Medikamente, Name des Arztes, Name des Apothekers, Apothekenadresse, Verschreibungsnummern, Nachfüllinformationen und/oder weitere patientenspezifischen Informationen. Entsprechende Informationen können in Schriftform auf den einzelnen Verpackungen angeordnet sein, um von dem Lesegerät erfasst zu werden. Jedoch können die Informationen auch oder zusätzlich mittels eines Codes, wie beispielsweise eines Strichcodes oder QR-Codes, Farben, Symbolen oder anderweitigen Kennzeichnungen, wie beispielsweise "Radio-Frequency Identification"-Transponder RFID-Tags), auf den einzelnen Verpackungseinheiten aufgebracht sein. Diese Kennzeichnung der Verpackungseinheiten dient der sicheren Handhabung der Medikamente, insbesondere um eine Fehlmedikation oder Manipulationen zu vermeiden.

Es besteht zusätzlich die Möglichkeit, den Blisterinhalt optisch zu prüfen, beispielsweise nach Größe, Form, Farbe, Anzahl der Tabletten. Ferner können über einen erweiterten Spektralbereich z.B. Infrarot IR) in einer Ausführungsform Tabletten näher identifiziert werden. Dadurch können, im unwahrscheinlichen Fall eines falschen Aufdrucks, Unstimmigkeiten erkannt und somit die Sicherheit weiter erhöht werden.

Bei dem Lesegerät kann es sich um eine oder mehrere Kameras oder Sensoren handeln, die es erlauben, Informationen auf den Verpackungseinheiten, wie oben dargestellt, zu erkennen und zu lesen. In einer bevorzugten Ausführungsform handelt es sich bei dem Lesegerät, das die Daten erfasst, um einen Barcodeleser, der vorzugsweise Strichcodes oder QR-Codes auf der Verpackungseinheit optisch mittels Rot- oder Infrarotlicht erfasst. Das Dekodiergerät, welches die ausgelesenen Informationen umwandelt, kann hierbei in das Lesegerät integriert sein oder extern in der Vereinzelungseinrichtung 8 angeordnet sein. Eine direkte Erfassung und Auswertung mittels eines Tabletcomputers bzw. Smartphones ist ebenfalls möglich. Aus Platzgründen wird eine integrierte Lösung hierbei jedoch bevorzugt. Die ermittelten Informationen werden im Anschluss an einen Rechner übermittelt, der einen Datenabgleich mit einer internen oder externen Datenbank bzw. mit im Speicher umfassten Informationen durchführt. Ein solcher Datenabgleich soll dazu dienen, dass eine personenbezogene Blisterverpackung an den entsprechenden Patienten um eine vorzugsweise spezielle Uhrzeit abgegeben wird.

Die Datenübermittlung zum Informationsabgleich kann mittels kabelgebundener oder bekannter kabelloser Technologien erfolgen. Beispiele für kabellose Übertragungsverbindungen umfassen Wireless-LAN, d.h. drahtlose lokale Netzwerke, oder Mobilfunk, wie beispielsweise LTE, 5G, GSM. Für die Datenübertragungsverfahren weist die erfindungsgemäße Einrichtung umfassend das Aufbewahrungsbehältnis 1 und die Vereinzelungseinrichtung 8 entsprechende Anschlüsse und Einheiten auf, die eine solche Übertragung ermöglichen. Solche Anschlüsse und Einheiten umfassen, sind jedoch nicht begrenzt auf, einen oder mehrere Netzwerkanschlüsse, wie beispielsweise eine RJ-45-Buchse, einen Anschluss für Lichtwellenleiter, Modemanschluss, ISDN-Karten-Anschluss, logische Netzwerkschnittstellen, Software und/oder drahtlose Netzwerkschnittstellen. Mithilfe der Datenübermittlung und des Informationsabgleichs wird es möglich Informationen, Aktualisierungen, Medikationen und Warnmeldungen zu empfangen und/oder zu versenden.

Die Vereinzelungseinrichtung 8 umfasst weiterhin in einer Ausführungsform mindestens einen Sender 11 und mindestens einen Empfänger 12, die die Position der Verpackungseinheiten innerhalb der Vereinzelungseinrichtung 8 bestimmen. Die Ermittlung eines bestimmten Ortes der Verpackungseinheiten in Bezug auf einen bestimmten Bezugspunkt, nämlich vorzugsweise den Schneidebereich eines Trennwerkzeugs, soll dazu dienen, dass die einzelne Verpackungseinheit oder eine bestimmte Anzahl von Verpackungseinheiten von der Aneinanderreihung der Vielzahl an Verpackungseinheiten an einer vorbestimmten Position getrennt wird. Bei den Elementen zur Positionsbestimmung handelt es sich vorzugsweise um Lichtschranken und/oder Kameras, oder ähnliche Sensoren, die entsprechende Formen an den Verpackungseinheiten oder zwischen den einzelnen Verpackungseinheiten erkennen. Entsprechende Erkennungszeichen an bzw. zwischen den Verpackungen sind beispielsweise Marker, Perforationen und/oder Schweißnähte. Eine entsprechende Erkennung kann, beispielsweise bei Lichtschranken, durch die Unterbrechung des Lichtstrahls erfolgen, die durch einen geeigneten Empfänger detektiert wird. Wird eine bestimmte Markierung, wie beispielsweise eine Schweißnaht oder das Ende der Verpackungseinheit detektiert, wird die Position an mindestens eine Trenneinrichtung 15 übermittelt.

Die mindestens eine Trenneinrichtung 15 dient der Trennung der einzelnen Verpackungseinheiten, vorzugsweise eines einzelnen Blisters von der Rolle an Blistern. Hierbei handelt es sich um eine Klinge, ein Messer, eine Schere und/oder ein mit Wärme arbeitendes Trennwerkzeug, das mit einem Gegenstück wie einer Gegenklinge oder einem Gegenstück mit einer definierten Schnittkante 19 (von beispielsweise 90°) zusammenwirkt, um einen entsprechenden Schnitt durchzuführen und eine Abgabe einer einzelnen oder einer bestimmten Anzahl an Verpackungen an den Patienten ermöglichen.

In einer bevorzugten Ausbildung wird eine Klinge um einen Drehpunkt geschwenkt und gleitet entlang einer Gegenkante, wodurch der Blister abgeschert wird. Alternativ kann die Klinge translatorisch bewegt werden und weist zur Gewährleistung einer schneidenden Trennbewegung eine guillotinenenmesserartige schräge Schneide auf.

Die Trenneinrichtung 15 muss nicht zwangsweise scharf sein. In einer Ausführungsform wird durch eine entsprechende Form des Niederhalters und der Trenneinrichtung 15 der Blister an der Perforation durch eine Zugbeanspruchung abgerissen. Bei der Zugspannung kann es sich durch ein Ziehen des Anwenders selbst handeln oder es kann durch ein zusätzliches Element eine solche aufgebaut werden. Eine weitere Möglichkeit des Abreißens des Blisters an der Perforation ist, dass der abzutrennende Beutel, beispielsweise durch einen Niederhalter 14 und ein mit diesem zusammenwirkendes Fixierelement, fixiert wird und die Fördereinrichtung 9 in die entgegengesetzte Richtung rückwärts läuft. Nach erfolgtem Abtrennen wird die Fixierung gelöst und der Beutel kann entnommen werden.

Um eine möglichst exakte Positionierung und/oder Trennung der Verpackungseinheiten zu ermöglichen, ist in einer Ausführungsform ein Niederhalter 14 umfasst, der die Verpackungseinheiten in einer bestimmten Position hält, vorzugsweise, bevor eine Trenneinrichtung 15 einen Schnitt durchführt. Der Niederhalter 14 kann hierbei derart konstruiert sein, dass die Verpackungseinheiten durch das Förderband direkt unter einen solchen geführt werden und somit gehalten werden. Alternativ kann der Niederhalter 14 auch durch einen Sensor, beispielsweise die oben genannte Lichtschranke, aktiviert werden und durch einen mechanischen oder elektrischen Motor in Richtung der mindestens einen Verpackungseinheit bewegt werden und diesen durch Druckkraft in einer bestimmten Positionierung halten. Es ist jedoch wichtig, dass die auf die Verpackungen einwirkende Kraft nicht zu hoch ist, sodass die darin befindlichen Medikamente nicht gequetscht oder zerstört werden. Der Niederhalter 14 besteht vorzugsweise aus einem Kunststoff, einem Metall, Holz und/oder einem Verbundstoff. Vorzugsweise umfasst der Niederhalter einen Aufnahmespalt 20 zur Aufnahme der Klinge der Trenneinrichtung 15, dessen einer Rand gleichzeitig die Schnittkante 19 darstellt, die mit der Klinge der Trennreinrichtung 15 zusammenwirkt.

In einer bevorzugten Ausführungsform wird die Positionierung der Trenneinrichtung 15 über einen Sensor abgefragt. Ist die Trenneinrichtung 15 noch ausgefahren, verbleibt der Niederhalter 14 in der aktiven Position, d.h. dieser drückt mit einer gewissen Kraft auf die Verpackungseinheit. Ist die Trenneinrichtung 15 eingefahren, d.h. in seiner inaktiven Position, so gibt der Niederhalter 14 die Verpackungseinheit frei, d.h. die auf die Verpackungseinheit einwirkende Kraft wird aufgehoben oder verringert.

Die Vereinzelungseinrichtung 8 und/oder das Aufbewahrungsbehältnis 1 weisen vorzugsweise ferner mindestens ein Display 13 zur Darstellung auf. Bei den entsprechenden Informationen kann es sich, wie bereits zuvor beschrieben, um Informationen auf der Verpackungseinheit handeln, beispielsweise um die Namen sowie Chargen der Medikamente, die Dosierung sowie weitere Informationen zur Verwendung, Patientendaten, Informationen bezüglich der Apotheke oder des Arztes und/oder um allgemeine Hinweise. Das Display 13 kann auch als Touchpad ausgestaltet sein oder kann Tasten und/oder Eingabegeräte umfassen, die es ermöglichen bestimmte Informationen zu sichten und/oder bestimmte Daten einzutragen. Alternativ kann das Display 13 auch ein externes Gerät sein, durch welches die Einrichtung bedient werden kann und über welches eine Kommunikation zu Dritten erfolgen kann. Diese externen Geräte sind beispielsweise Laptops, Tablets, Mobiltelefone, PC oder Smartphones, jedoch ist diese Auflistung nicht begrenzt auf die aufgelisteten. Neben den zuvor genannten Informationen können zusätzlich durch das Display 13, das externe Gerät oder Leuchten an der Einrichtung Benachrichtigungen oder Warnmeldungen als visueller Alarm angezeigt werden, die den Patienten an die Einnahme einer Medikation erinnern oder einen Fehler sichtbar machen.

Zusätzlich zu den visuellen Mitteln können auch akustische und/oder taktile Mitteilungen an der Einrichtung oder den externen Geräten eingesetzt werden, um den Patienten oder eine andere Person zu erinnern oder zu warnen. Bei den externen Geräten kann es sich neben akustischen Signalen und taktilen Vibrationen um Textbenachrichtigungen, E-Mail-Benachrichtigungen, Anrufe oder ähnliches handeln.

In einer Ausführungsform betrifft die vorliegende Erfindung auch ein Verfahren zur Ausgabe einer bestimmten Verpackungseinheit oder mehrerer Verpackungseinheiten an einen Patienten. Hierbei wird eine Einrichtung wie zuvor beschrieben verwendet.

In einem solchen Verfahren wird ein zuvor beschriebenes Aufbewahrungsbehältnis 1, in welchem zusammenhängende Verpackungseinheiten aufgerollt gelagert sind mit einer zuvor beschriebenen Vereinzelungseinrichtung 8 in Kontakt gebracht, wobei eine Vorderseite des Aufbewahrungsbehältnisses 1 mit einer Aufnahmeseite der Vereinzelungseinrichtung 8 interagiert. Die Befüllung des Aufbewahrungsbehältnisses 1 erfolgt vorzugsweise durch eine mechanische Vorrichtung, die ein Magazin mit den aufgerollten Verpackungseinheiten in das Aufbewahrungsbehältnis 1 einführt. Die Vorderseite des Aufbewahrungsbehältnisses 1 weist hierbei eine Auflage 7 für die Verpackungseinheiten auf und eine Öffnung, die eine Abgabe der Verpackungseinheiten aus dem Aufbewahrungsbehältnis 1 ermöglicht. Die Aufnahmeseite der Vereinzelungseinrichtung 8, die mit der Auflage 7 und den Verpackungseinheiten interagiert, weist eine Vereinzelungseinrichtung 8 auf, die in der Lage ist, die Verpackungseinheiten aus dem Aufbewahrungsbehältnis 1 zu ziehen. Durch das In-Kontakt-bringen der beiden Öffnungen wird ein Hebel 10 oder ein entsprechendes Element an der Vereinzelungseinrichtung 8 durch eine am Aufbewahrungsbehältnis befindliche Rampe 4 in seiner Position verändert, nämlich indem der Hebel 10 nach oben gedrückt wird. Durch die weitere Annäherung des Aufbewahrungsbehältnisses 1 mit der Vereinzelungseinrichtung 8 wird eine Auflage 7 des Aufbewahrungsbehältnisses unter den Förderriemen geschoben. Gelangt das Aufbewahrungsbehältnis 1 in die Nähe der Vereinzelungseinrichtung 8 in die sogenannte Schwenkposition, die beispielsweise in der Fig. 2b dargestellt ist, so beginnt sich der Deckel 2 des Aufbewahrungsbehältnisses zu öffnen.

Erreichen das Aufbewahrungsbehältnis 1 und die Vereinzelungseinrichtung 8 eine Endposition, wie beispielsweise der Fig. 2c zu entnehmen ist, so stellt ein Element zur Positionserkennung 5 eine solche Endposition fest. Das Element zur Positionserkennung 5, bei dem es sich vorzugsweise um einen druckempfindlichen Sensor, Schalter oder Taster handelt, wird hierbei mit einer Kraft beaufschlagt, wodurch die Position mittels einer Steuerung an das Aufbewahrungsbehältnis 1 selbst, sowie an die Vereinzelungseinrichtung 8 übermittelt wird. Durch eine entsprechende Informationsweitergabe wird die weitere Öffnung des Deckels 2 des Aufbewahrungsbehältnisses gestoppt. Ferner wird dem Antrieb 9 in der Vereinzelungseinrichtung 8 signalisiert, sich zu senken. Der federgelagerte Riementrieb wird hierbei auf die Auflage 7 des Aufbewahrungsbehältnisses gesenkt, auf welcher eine Verpackungseinheit angeordnet ist. Nach erfolgter Absenkung wird der Antrieb 9 aktiviert, wodurch die Verpackungseinheiten aus dem Aufbewahrungsbehältnis 1 in die Fördereinheit transportiert werden. Ein entsprechender Transport erfolgt hierbei von einer Eingangsöffnung zu einer Ausgangsöffnung innerhalb der Vereinzelungseinrichtung 8.

Um eine exakte Positionierung der Verpackungseinheit innerhalb der Vereinzelungseinrichtung 8 bestimmen zu können, ist mindestens eine Sensoreinheit innerhalb der Vereinzelungseinrichtung 8 angeordnet, die den Ort der Verpackungseinheiten bzw. der Verpackungseinheit in Bezug auf einen bestimmten Bezugspunkt bestimmt. Erreicht die Verpackungseinheit einen solchen Bezugspunkt, wie vorzugsweise den Schneidebereich, so wird in einer Ausführungsform die Verpackungseinheit an dieser Position fixiert. Eine solche Fixierung erfolgt vorzugsweise über einen Niederhalter 14, der die Verpackungseinheit in einer bestimmten Position hält.

Anschließend werden eine Verpackungseinheit oder mehrere Einheiten von den zusammenhängenden Verpackungseinheiten getrennt und an der Ausgabeöffnung der Vereinzelungseinrichtung 16 ausgegeben.

Ein solches Verfahren soll erreichen, dass die Abgabe der einzelnen Verpackungseinheiten geregelt erfolgt und Manipulationen verhindert werden.

In einer Ausführungsform erfolgt zwischen dem Transport der Verpackungseinheit von einer Eingangsöffnung zu einer Ausgangsöffnung innerhalb der Vereinzelungseinrichtung 8 und der Bestimmung der Position der Verpackungseinheit innerhalb der Vereinzelungseinrichtung 8 eine Überprüfung von Daten auf der Verpackungseinheit. Entsprechende Daten auf der Verpackungseinheit umfassen, sind jedoch nicht beschränkt auf den Namen des Patienten, Adresse des Patienten, Datum der Verpackung, Datum der Herstellung, Verfallsdatum des bzw. der Medikamente, Datum der Verabreichung, Uhrzeit der Verabreichung, Anweisungen zur Einnahme des Medikaments, Warnhinweise, Verpackungsinhalt, Dosierung der Medikamente, Name des Arztes, Name des Apothekers, Apothekenadresse, Verschreibungsnummern, Nachfüllinformationen und/oder weitere patientenspezifische Informationen, wie zuvor zur Vorrichtung beschrieben. Die Daten können neben der Überprüfung und dem Abgleich mit internen und/oder externen Datenbanken auch an ein Display 13 oder externe Geräte zur Darstellung, Warnung und/oder Bearbeitung übermittelt werden. Die Datenübermittlung zum Informationsaustausch kann hierbei mittels kabelgebundener oder kabelloser Technologien erfolgen, wie beispielsweise drahtlose lokale Netzwerke, oder Mobilfunk, wie beispielsweise Long Term Evolution LTE, 5G, Universal Mobile Telecommunications System UMTS und/oder Global System for Mobile Communications GSM.

In einer Ausführungsform umfassen die Einrichtung sowie das Verfahren eine Steuerung, einen Mikroprozessor bzw. einen Steuerungsmechanismus, der die einzelnen zusammenwirkenden Elemente und deren Aufgaben steuert. Der Steuermechanismus überträgt hierzu die Informationen von den einzelnen Bauteilen innerhalb der Einrichtung bzw. von den Informationen der Verpackungseinheiten sowie die Positionierung der einzelnen Elemente der Einrichtung vorzugsweise an einen Rechner, der diese auswertet, in einem Speicher speichert und/oder an eine weitere Einheit zur Bearbeitung, Darstellung und/oder Wirkung weitergibt. In einer Ausführungsform wird der Patient, die Apotheke oder der behandelnde Arzt darüber informiert, dass das Ende des einer Verpackungseinheit bzw. des Magazins umfassend die Verpackungseinheiten erreicht wurde oder in Kürze erreicht wird. Eine solche Informationsabgabe erfolgt hierbei durch die Überprüfung sowie Übermittlung mittels kabelloser oder kabelgebundener Datenübertragung an ein entsprechendes Gerät.

Zur Steuerung des Aufbewahrungsbehältnisses 1, der Vereinzelungseinrichtung 8 und/oder zur Daten- und Informationenweitergabe umfasst das Aufbewahrungsbehältnis 1, die Vereinzelungseinrichtung 8 und/oder ein externes Element, welches mit einem oder beiden Elementen verbunden ist, ein Rechenmodul, wie beispielsweise einen Computer. Daher betrifft vorliegende Erfindung weiterhin ein Computerprogramm zum Durchführen eines zuvor beschriebenen Verfahrens, wenn das Computerprogramm auf einem Computer ausgeführt wird.

Beim Implementieren des Verfahrens werden zumindest einige der zu dem Verfahren gehörenden Schritte durch einen Prozessor durch Ausführung von Anweisungen durchgeführt. In einer weiteren Ausführungsform können Anweisungen oder ein Teil der Anweisungen zur Ausführung des beschriebenen Verfahrens und/oder zur Implementierung des beschriebenen Verfahrens in ein System auf einem computerlesbaren Datenträger gespeichert werden.

Die Anweisungen können auch in einem von einem Computer, oder entsprechenden Gerät verwendbaren oder von solchen lesbaren Speicher gespeichert sein, der einen Computer, ein ähnliches Gerät oder eine andere programmierbare Datenverarbeitungsvorrichtung anweisen kann, auf eine bestimmte Weise zu wirken, sodass die in dem von einem Computer verwendbaren oder von einem Computer lesbaren Speicher gespeicherten Anweisungen erzeugt werden. Die Anweisungen können in einer Ausführungsform auch auf andere Geräte geladen werden, um eine Reihe von Arbeitsschritten zu veranlassen.

Die Einrichtung der vorliegenden Erfindung kann in diesem Zusammenhang daher eine Vielzahl von identischen und/oder unterschiedlichen Modulen umfassen. Als Module werden auch "Komponenten" oder "Funktionseinheiten" bezeichnet. Darüber hinaus können Module und/oder Komponenten auch "computerausgeführt" und/oder "computerimplementiert" sein. Die Module werden hierbei im Rahmen eines Computersystems implementiert, das typischerweise einen Prozessor und einen Speicher umfasst. Im Allgemeinen ist ein Modul eine Komponente eines Systems, das bestimmte Operationen für die Implementierung einer bestimmten Funktionalität ausführt. Beispiele für Funktionalitäten umfassen den Empfang von Messwerten, wie beispielsweise Informationen vom Lesegerät sowie patientenbezogene Daten aus beispielsweise einer Datenbank. Die Module können jedoch auch weitere, in den obigen Ausführungsformen zum Verfahren, beschriebene Funktionalitäten besitzen. Durch den Abgleich mit einer Datenbank ist es zusätzlich möglich, die Ausgabe bzw. die Einrichtung zur Abgabe einer Verpackungseinheit zu sperren. Dies kann beispielsweise bei Medikamentenrückruf oder einer nachträglich herausgefundenen Fehlmedikation notwendig sein.

Der Begriff "Modul" umfasst hier eine greifbare Entität, die physisch konstruiert ist, dauerhaft konfiguriert ist (z. B. festverdrahtet) oder temporär konfiguriert ist (z. B. programmiert), um auf eine bestimmte Weise zu operieren oder bestimmte hierin beschriebene Operationen auszuführen. In Ausführungsformen, in denen die Module temporär konfiguriert, beispielsweise programmiert, sind, muss nicht jedes Modul zu jedem Zeitpunkt konfiguriert oder instanziiert werden. Beispielsweise kann ein allgemeiner Prozessor derart konfiguriert sein, dass er verschiedene Module zu unterschiedlichen Zeiten ausführt. In einigen Ausführungsformen implementiert ein Prozessor ein Modul durch Ausführung von Anweisungen, die zumindest ein Teil der Funktionalität des Moduls implementieren. Optional kann ein Speicher die Anweisungen, beispielsweise als Computer-Code, speichern, die durch den Prozessor gelesen und verarbeitet werden und bewirken, dass der Prozessor zumindest einige an der Implementierung der Funktionalität des Moduls beteiligte Operationen durchführt.

Zusätzlich oder alternativ kann ein Speicher, der ein oder mehrere Speichergeräte umfassen kann, in einer Ausführungsform Daten speichern, die durch den Prozessor gelesen und verarbeitet werden, um zumindest einen Teil der Funktionalität des Moduls zu implementieren. Der Speicher kann in einer weiteren Ausführungsform ein oder mehrere Hardware-Elemente umfassen, die Informationen speichern können, die für einen Prozessor zugänglich sind.

Der mindestens eine Prozessor führt in einer Ausführungsform auf dem Speicher gespeicherte Anweisungen aus, die an der Implementierung der Funktionalität eines bestimmten Moduls beteiligte Operationen durchführen. Der mindestens eine Prozessor kann zudem derart operieren, dass die Leistung der relevanten Operationen in einer Umgebung mit "Cloud-Computing" oder als "Software-as-a-Service" (SaaS) unterstützt wird. Zum Beispiel können zumindest einige der an der Implementierung eines Moduls beteiligten Operationen durch eine Gruppe von Computern durchgeführt werden, die über ein Netzwerk, wie beispielsweise das Internet und/oder über eine oder mehrere entsprechende Schnittstellen, wie Anwendungsprogramm-Schnittstellen (API) zugänglich sind. Optional können einige der Module in einer verteilten Weise zwischen mehreren Prozessoren ausgeführt werden. Der mindestens eine Prozessor kann sich in einer Ausführungsform an einem geographischen Ort befinden oder auf mehrere geographische Orte verteilt sein. Optional können Informationen zu Patienten und/oder der Medikation, wie sie in der Verpackungseinheit enthalten ist, auch teilweise oder vollständig auf Prozessoren ausgeführt werden, die zu anderen Geräten gehören. Diese Geräte sind beispielsweise Laptops, Tablets, Smartphones, jedoch ist diese Auflistung nicht begrenzt auf die aufgelisteten, sondern kann jedes bekannte Gerät umfassen, das eine Informationsdarstellung und/oder einen Datenabgleich ermöglicht. Dementsprechend müssen die Geräte nicht explizit am Ort der Vorrichtung gelegen sein. Weiterhin können Daten in einer Ausführungsform zu Cloud-basierten Servern hochgeladen werden. In einigen Ausführungsformen können Module anderen Modulen Informationen bereitstellen und/oder Informationen von anderen Modulen empfangen. Entsprechend können solche Module als kommunikativ gekoppelt gelten. Wenn mehrere solcher Module gleichzeitig vorhanden sind, können Kommunikationen durch Signalübertragung erreicht werden. In Ausführungsformen, in denen Module zu unterschiedlichen Zeiten konfiguriert oder instanziiert werden, können Kommunikationen zwischen solchen Modulen beispielsweise durch das Speichern und Abrufen von Informationen in Speicherstrukturen, auf die mehrere Module zugreifen können, erreicht werden. In einer Ausführungsform kann ein Modul eine Operation ausführen und den Output dieser Operation auf einem Speichergerät speichern, mit dem es kommunikativ gekoppelt ist. Ein anderes Modul kann dann zu einem späteren Zeitpunkt auf das Speichergerät zugreifen, um das gespeicherte Output abzurufen und zu verarbeiten.

Im Hinblick auf die Funktionalität und Wirkung eines oder aller Programmmodule ist festzuhalten, dass diese auch unter Verwendung diskreter Hardwarekomponenten, einer oder mehrerer anwendungsspezifischer integrierter Schaltungen (ASICs), eines programmierten digitalen Signalprozessors und/oder Mikrocontrollers implementiert werden können. Hierbei können die Programmcodes bzw. der Programmcode auch vollständig auf einem einzelnen Prozessor, auf mehreren Prozessoren als eigenständiges Softwarepaket und/oder als Teil eines anderen Softwarepakets ausgeführt werden. Gleiches gilt für die Ausführung auf den Vorrichtungen, Die Programme bzw. Programmcodes können vollständig oder teilweise auf einem Gerät und teilweise auf einem anderen Gerät ausgeführt werden. Eine entsprechende Kommunikation zwischen den Geräten erfolgt hierbei vorzugsweise über ein drahtgebundenes, drahtloses lokales Netzwerk LAN, ein Weitverkehrsnetz (WAN) und/oder eine Verbindung zu einem externen Computer oder Server mittels Internet.

Diese und andere Ausführungsformen der vorliegenden Erfindung werden in der Beschreibung und den Beispielen offenbart und sind durch diese umfasst. Weitere Literatur über bekannte Materialien, Verfahren und Anwendungen, die in Übereinstimmung mit der vorliegenden Erfindung verwendet werden können, können aus öffentlichen Bibliotheken und Datenbanken, beispielsweise unter Verwendung elektronischer Geräte aufgerufen werden. Ein vollständigeres Verständnis der Erfindung kann durch Bezugnahme auf die folgenden Beispiele erhalten werden, die zum Zweck der Illustration bereitgestellt wurden und den Umfang der Erfindung nicht beschränken sollen.

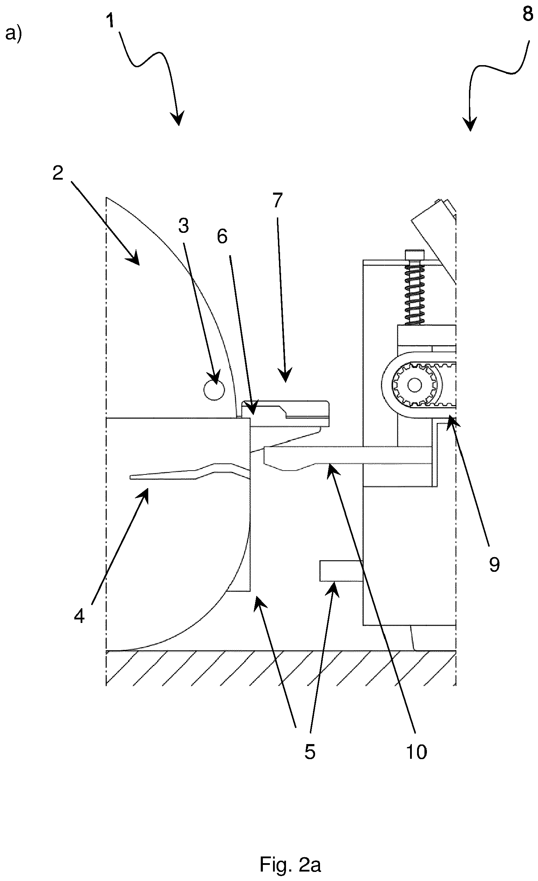

In der Fig. 2a ist eine Anfangsposition der miteinander agierenden Einheiten, nämlich des Aufbewahrungsbehältnisses 1 mit der Vereinzelungseinrichtung 8 in einer bevorzugten Ausführungsform, dargestellt. Wie der Figur zu entnehmen ist, zeigt die Auflage 7, die sich an der Vorderseite des Aufbewahrungsbehältnisses 1 befindet, in Richtung der Eingangsöffnung der Vereinzelungseinrichtung 8, an welcher mindestens ein Hebel 10 seitlich, nach vorne herausragend angeordnet ist. In der Anfangsposition ist der Deckel 2 des Aufbewahrungsbehältnisses geschlossen und der Antrieb 9 der Vereinzelungseinrichtung ist nicht aktiv.

Im Seitenbereich des Aufbewahrungsbehältnisses 1, vorzugsweise an beiden Seiten, ist eine Art Rampe angeordnet. Diese Rampe 4 interagiert mit dem Hebel 10 der Vereinzelungseinrichtung derart, dass der Hebel 10 bei einer Zusammenführung der beiden Elemente über eine Wölbung in der Rampe 4 geschoben wird. Um ein Verrutschen zwischen Hebel 10 und Rampe 4 während der Interaktion zu verhindern, umfasst der Hebel eine Hervorhebung, die in Richtung der Rampe 4, also nach unten zeigt. Beim Zusammenschieben des Aufbewahrungsbehältnisses 1 mit der Vereinzelungseinrichtung 8 verhaken sich die beiden Elemente, nämlich Haken und Rampe 4 miteinander, wie beispielsweise der Fig. 2c zu entnehmen ist.

Durch das Einschieben des Aufbewahrungsbehältnisses 1 drückt daher die Rampe 4 den Hebel 10 nach oben, zur Oberseite der Vereinzelungseinrichtung 8, wodurch sich die Auflage 7 unter den Antrieb 9 schiebt und den federgelagerten Antrieb 9 leicht nach oben anhebt, wie der Fig. 2b zu entnehmen ist. In dieser Schwenkposition in Fig. 2b beginnt sich der Deckel 2 des Aufbewahrungsbehältnisses zu öffnen. Gelangt das Aufbewahrungsbehältnis 1 in die Endposition, wie der Fig. 2c zu entnehmen ist, senkt sich der federgelagerte Antrieb 9 auf die Auflage 7 ab. Der Deckel 2 des Aufbewahrungsbehältnisses erlangt die geöffnete Position.

Die Endposition des Aufbewahrungsbehältnisses 1 und der Vereinzelungseinrichtung 8 zueinander wird in einer bevorzugten Ausführungsform durch einen Taster erkannt. Das daraus generierte Signal wird an eine Steuerung übertragen, die den Einzug des Förderbandes aktiviert, wodurch ein Vorschub aktiviert wird und die Verpackungseinheiten aus dem Aufbewahrungsbehältnis 1 in die Vereinzelungseinrichtung 8 gezogen werden. Ein gummierter Riementrieb transportiert die zusammenhängenden Verpackungseinheiten hierbei von der Eingangsöffnung zu einer Ausgangsöffnung innerhalb der Vereinzelungseinrichtung 8. Eine Strommessung überprüft beim Einzug der Verpackungseinheiten vorzugsweise die Schwergängigkeit und/oder eine mögliche Blockierung des Einzuges.

Die Verpackungseinheiten werden innerhalb der Vereinzelungseinrichtung 8 auf ihre Zusammensetzung und Darreichung kontrolliert, indem ein Lesegerät, vorzugsweise Barcodescanner, eine Kamera oder Ähnliches, die an den Verpackungseinheiten angeordneten Codierungen, Markern, Symbolen oder andere Aufbringungen aufnimmt und mittels einer Recheneinheit eine Überprüfung der Daten mit internen und/oder externen Datenbanken durchführt.

Im weiteren Verlauf wird die Position der Verpackungseinheit in Bezug auf die Trenneinrichtung 15 innerhalb der Fördereinheit ermittelt. Hierzu sind in einer bevorzugten Ausführungsform drei Lichtschranken in der Einheit angeordnet, die vorzugsweise zwei an den Verpackungseinheiten angeordnete Codes oder Marker sowie die Verschweißungen zwischen den einzelnen Verpackungseinheiten erkennen. Durch diese dreifache Redundanz soll eine Fehlpositionierung vermieden werden.

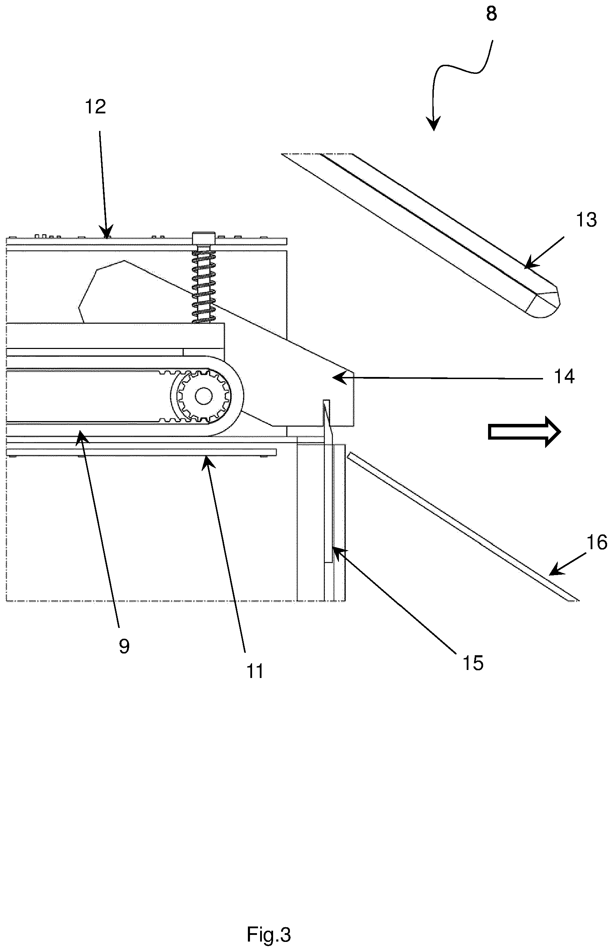

Ist eine Positionsbestimmung der Verpackungseinheiten erfolgt, schwenkt ein Niederhalter 14 nach unten und hält die Verpackungseinheiten in einer bestimmten Position, wie beispielsweise der Fig. 3 zu entnehmen ist. Der Anpressdruck des Niederhalters 14 im Zusammenhang mit den Verpackungseinheiten kann hierbei durch eine Strommessung innerhalb Motors des Niederhalters 14 variieren. Ein entsprechender Druck kann durch eine entsprechende Steuerung bzw. einen Rechner, der einen solchen berechnet, ausgegeben werden.

Hält der Niederhalter 14 die Verpackungseinheiten in einer bestimmten Position, wird ein Schneidwerkzeug oder eine andere Trenneinrichtung 15, wie beispielsweise ein Messer (Fig. 3), aktiviert. Dieses wird aus einem unteren Bereich der Vereinzelungseinrichtung 8 vertikal nach oben geschoben und trennt die aneinanderhängenden Verpackungseinheiten oder eine Verpackungseinheit ab.

Die Verpackungseinheit oder die Verpackungseinheiten werden frei und werden durch eine Ausgabeöffnung der Vereinzelungseinrichtung 16, die beispielsweise auch eine Rutsche aufweisen kann, abgegeben und können entnommen werden.

Während des gesamten Verfahrens werden zwischenzeitlich Sicherheitsabfragen an die Steuerung und/oder die einzelnen Elemente gestellt, um eine sichere Abgabe der Verpackungseinheiten zu ermöglichen. Entsprechende Sicherheitsabfragen, die durch entsprechende Sensoren und Positionierungen der Einheiten überprüft werden, sind beispielsweise:
"Ist das Aufbewahrungsbehältnis eingeschoben?", "In welcher Position befindet sich der Niederhalter?", "In welcher Position befindet sich das Trennwerkzeug?"

Wie der Fig. 4 zu entnehmen ist, weist das Aufbewahrungsbehältnis 1 an der Vorderseite eine Auflage 7 auf, auf der die Verpackungseinheiten geführt werden. Diese Führung umfasst ferner in den Seitenbereichen Hervorhebungen auf, die eine undefinierte Bewegung der Verpackungseinheiten durch die Zwangsführung 6 minimieren sollen.

Die Auflage 7 ist vorzugsweise starr angeordnet und ermöglicht somit die Ausübung eines Druckes gegen den Antrieb 9 der Vereinzelungseinrichtung. Durch das Zusammenschieben des Aufbewahrungsbehältnisses 1 mit der Vereinzelungseinrichtung 8 wird durch die Anlauframpe 4 der federgelagerte Riementrieb angehoben, so dass sich die Auflage 7 unter den Förderriemen der Vereinzelungseinrichtung schieben kann, wie beispielsweise der Fig. 2b zu entnehmen ist.

An beiden Seitenbereichen, vorzugsweise im Bereich des Deckels 2, weist das Aufbewahrungsbehältnis Zylinder 3 auf, die eine automatisierte Öffnung des Deckels 2 ermöglichen, wie der Fig. 4 zu entnehmen ist.

An der unteren Vorderseite des Gehäuses des Aufbewahrungsbehältnisses 1 unterhalb der Auflage 7 ist eine Kontaktfläche für einen Positionstaster angeordnet, die beim Zusammenführen des Aufbewahrungsbehältnisses 1 mit der Vereinzelungseinrichtung 8 mit einem an diesem angeordneten Positionstaster interagiert und die Bestimmung der Endpositionierung ermöglicht.

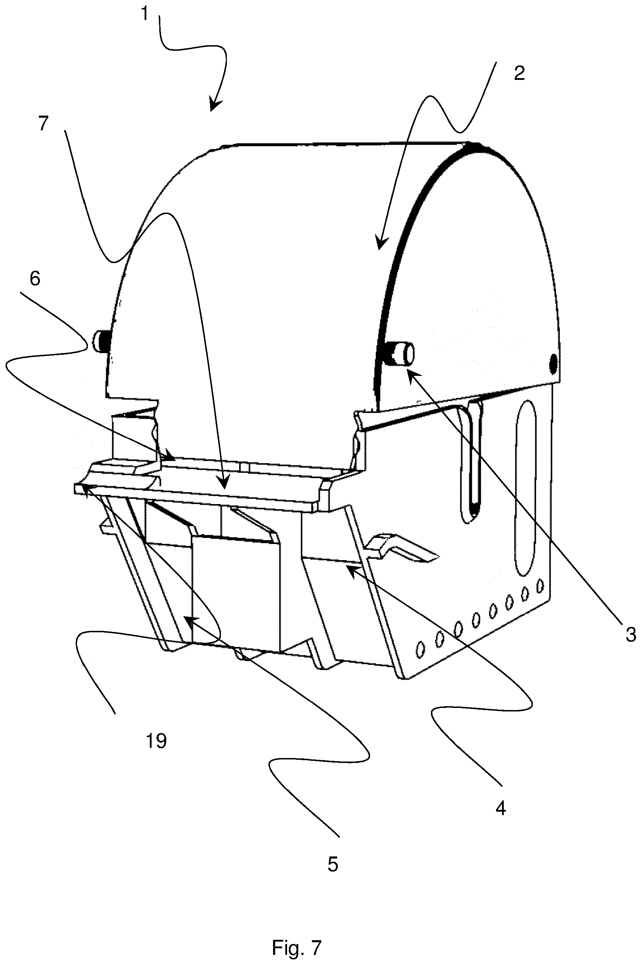

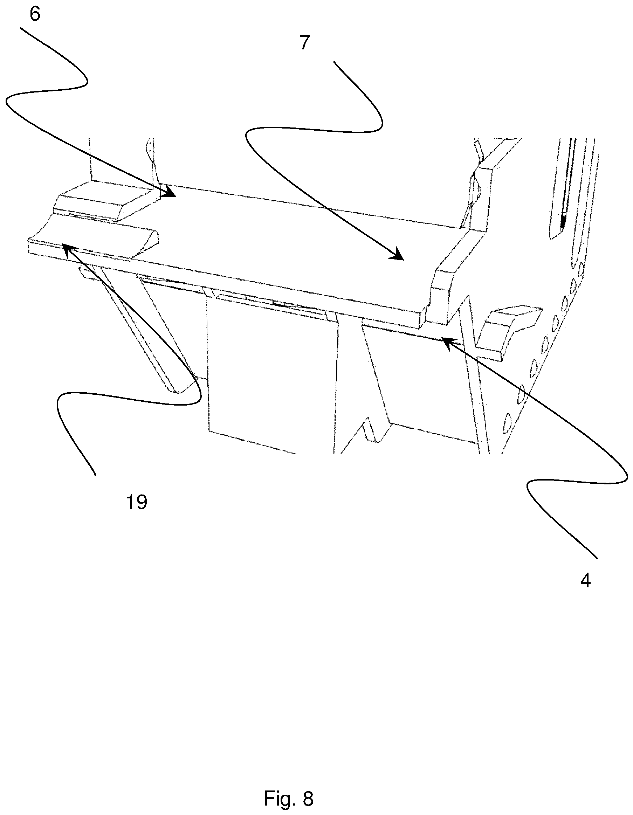

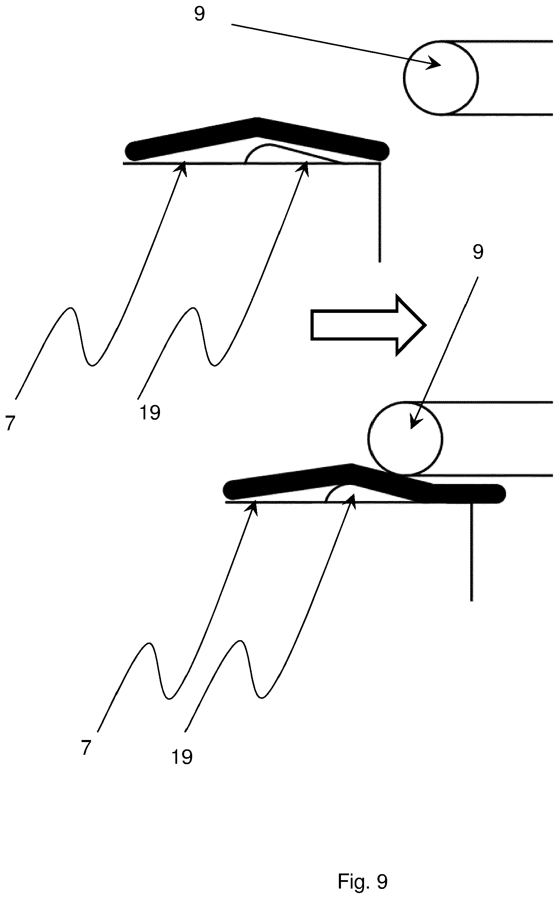

In Figur 7 und 8 ist eine perspektivische Darstellung eines Aufbewahrungsbehältnisses 1 zu sehen, das eine Erhebung 19 auf der Auflage 7 umfasst, auf welcher die schlauchförmige Verpackungseinheit 21 aufliegen kann. In Figur 9 ist schematisch der Vorgang der Kopplung von Aufbewahrungsbehältnis 1 und Vereinzelungseinrichtung 8 dargestellt, bei welcher vor der Kopplung (Fig. 9 oben) die schlauchförmige Verpackungseinheit 21 von der Erhebung 7 nach oben gedrückt ist. Die Fördereinrichtung 9 gelangt bei der Bewegung von Aufbewahrungsbehältnis 1 und Vereinzelungseinrichtung 8 gegeneinander in Eingriff mit dem Endbereich der vordersten Verpackungseinheit 21, die zwischen Fördereinrichtung 9 und Erhebung 19 festgehalten wird. Setzt sich die Fördereinrichtung 9 dann in Bewegung, wird die Verpackungseinheit 21 mitgenommen und zur Trenneinrichtung 15 (Fig. 1) geführt. Auf diese Weise lässt sich ein automatisierter Einzug der Verpackungseinheiten 21 in die Fördereinrichtung 9 realisieren, die außer dem manuellen In-Kontakt-Bringen von Aufbewahrungsbehältnis 1 und Vereinzelungseinrichtung 8 keinen manuellen Eingriff mehr benötigt.

### Bezugszeichenliste

1 Aufbewahrungsbehältnis
2 Deckel
3 Zylinder (für automatisiertes Öffnen)
4 Rampe (für Hebel-Riemenantrieb)
5 Element zur Positionserkennung (Positionstaster)
6 Zwangsführung
7 Auflage
8 Vereinzelungseinrichtung
9 Fördereinrichtung
10 Hebel (-Riemenantrieb)
11 Sender
12 Empfänger
13 Display
14 Niederhalter
15 Trenneinrichtung
16 Verpackungseinheitausgabe
17 Öffnungsmechanismus
18 Kontakt
19 Schnittkante
20 Aufnahmespalt
21 schlauchförmige Verpackungseinheit

## Patentansprüche

1. Einrichtung zur Abgabe einer Verpackungseinheit umfassend ein Aufbewahrungsbehältnis (1) mit einem Deckel (2) zur Aufnahme einer Aneinanderreihung einer Vielzahl schlauchförmiger Verpackungseinheiten und eine davon getrennte Vereinzelungseinrichtung (8) mit einer Fördereinrichtung (9), einer Trenneinrichtung (15) und einer Verpackungseinheitausgabe (16), die mit dem Aufbewahrungsbehältnis (1) in Eingriff bringbar ist, **dadurch gekennzeichnet, dass** der Deckel (2) durch das Ineingriffbringen zwischen Aufbewahrungsbehältnis (1) und Vereinzelungseinrichtung (8) automatisiert geöffnet wird und eine vorderste Verpackungseinheit (21) mit der Fördereinrichtung (9) in Eingriff gebracht und von dieser zur Verpackungseinheitausgabe (16) befördert wird.

2. Einrichtung nach Anspruch 1, wobei das Aufbewahrungsbehältnis (1)
a) an einer Ausgabeöffnung eine Auflage (7) mit Zwangsführung (6) für die Aneinanderreihung der Verpackungseinheiten aufweist; und/oder
b) ein Element zur Positionserkennung (5) umfasst, welches die Position zwischen Aufbewahrungsbehältnis (1) und Vereinzelungseinrichtung (8) bestimmt.

3. Einrichtung nach Anspruch 1 oder 2, wobei das Aufbewahrungsbehältnis (1) eine Anlauframpe (4) umfasst, welche beim In-Kontakt-bringen des Aufbewahrungsbehältnisses (1) mit der Vereinzelungseinrichtung (8) mit einem Hebel (10) der Vereinzelungseinrichtung (8) in Kontakt gelangt, wodurch eine Auflage (7) des Aufbewahrungsbehältnisses unter eine Fördereinrichtung (9) der Vereinzelungseinrichtung (8) geschoben wird.

4. Einrichtung nach einem der Ansprüche 1 bis 3, wobei die Vereinzelungseinrichtung (8) ein Förderelement umfasst, das die Aneinanderreihung der Verpackungseinheiten von einer Eingabeöffnung in der Vereinzelungseinrichtung (8) zur Ausgabeöffnung der Vereinzelungseinrichtung (16) transportiert, wobei die Fördereinrichtung (9) einen federgelagerter Hebel-Riemenantrieb (10) umfasst, der mit einer Anlauframpe (4) im Aufbewahrungsbehältnis interagiert.

5. Einrichtung nach einem der Ansprüche 1 bis 4, wobei die Vereinzelungseinrichtung (8) ein Lesegerät umfasst, das Informationen der Verpackungseinheiten an einen Rechner überträgt.

6. Einrichtung nach einem der Ansprüche 1 bis 5, wobei die Vereinzelungseinrichtung (8) mindestens einen Sender (11) und mindestens einen Empfänger (12) umfasst, die die Positionen der Verpackungseinheiten innerhalb der Vereinzelungseinrichtung (8) bestimmen.

7. Einrichtung nach einem der Ansprüche 1 bis 6, wobei die Vereinzelungseinrichtung (8)
a) mindestens ein Display (13) zur Darstellung umfasst;
b) mindestens eine Trenneinrichtung (15) zur Trennung der einzelnen Verpackungseinheiten (21) umfasst;
c) ein Element zur Positionserkennung (5) umfasst; und/oder
d) einen Niederhalter (14) umfasst, der die Verpackungseinheiten in einer bestimmten Position hält.

8. Einrichtung nach einem der Ansprüche 1 bis 7, wobei der Deckel (2) des Aufbewahrungsbehältnisses (1) eine Verriegelung umfasst, um eine Manipulation darin enthaltener Verpackungseinheiten (21) zu verhindern, wobei vorzugsweise die Verriegelung mittels Schlüssel oder ähnlichem Gegenstand, einer Zahlenkombination, biometrischer Identifizierung, Smartphone und/oder anderer Identifizierungen erfolgt.

9. Einrichtung nach einem der Ansprüche 2 bis 8, wobei die Zwangsführung (6) Führungselemente umfasst, die die seitliche Bewegung der Verpackungseinheiten (21) minimiert oder unterbindet.

10. Einrichtung nach einem der Ansprüche 2 bis 9, wobei das Element zur Positionserkennung (5) eine Kontaktfläche am Aufbewahrungsbehältnis (1) und einen Positionstaster an der Vereinzelungseinrichtung (8) umfasst.

11. Verfahren zur Ausgabe einer bestimmten Verpackungseinheit an einen Patienten mit einer Einrichtung nach einem der Ansprüche 1 bis 10, wobei das Verfahren folgende Schritte umfasst:
a) In-Kontakt-bringen eines Aufbewahrungsbehältnisses (1) mit einer Vereinzelungseinrichtung (8), wodurch eine Anlauframpe (4) des Aufbewahrungsbehältnisses (1) einen Hebel-Riementrieb (10) der Vereinzelungseinrichtung nach oben schiebt und eine Auflage (7) des Aufbewahrungsbehältnisses (1) sich unter den Förderriemen schiebt;
b) Öffnen eines Deckels (2) des Aufbewahrungsbehältnisses (1);
c) Feststellung einer Endposition zwischen Aufbewahrungsbehältnis (1) und Vereinzelungseinrichtung (8);
d) Absenkung eines federgelagerten Riementriebs auf die Auflage (7) und Aktivierung der Fördereinrichtung (9);
e) Transport der Verpackungseinheit (21) von einer Eingangsöffnung zu einer Verpackungseinheitausgabe (16) innerhalb der Vereinzelungseinrichtung (8);
f) Bestimmung der Position der Verpackungseinheit (21) innerhalb der Vereinzelungseinrichtung (8);
g) Fixierung der Verpackungseinheit (21) in einer bestimmten Position; und
h) Trennung einer Verpackungseinheit (21) von der Aneinanderreihung.

12. Verfahren nach Anspruch 11, wobei zwischen dem Transport der Verpackungseinheit (21) von einer Eingangsöffnung zur Verpackungseinheitausgabe (16) innerhalb der Vereinzelungseinrichtung (8) (Schritt e) und der Bestimmung der Position der Verpackungseinheit (21) innerhalb der Vereinzelungseinrichtung (8) (Schritt f) eine Überprüfung von Daten auf der Verpackungseinheit erfolgt.

13. Verfahren nach Anspruch 11 oder 12, wobei ein Verschluss des Deckels (2) des Aufbewahrungsbehältnisses (1) zur Bestückung mit Verpackungseinheiten (21) mittels Schlüssel oder eines ähnlichen Gegenstands, einer Zahlenkombination, biometrischer Identifizierung, eines Smartphones und/oder anderer Identifizierungen geöffnet wird.

14. Verfahren nach einem der Ansprüche 11 bis 13, wobei die Überprüfung von Daten den Abgleich mit internen und/oder externen Datenbanken umfasst, vorzugsweise wobei Informationen zum Verpackungsinhalt, dem Patienten, den Darreichungsformen und/oder weiteren Informationen auf einem Display (13) der Vereinzelungseinrichtung (8) oder eines externen Geräts wiedergegeben werden.

## Claims

1. Device for dispensing a package unit, comprising a storage container (1) with a cover (2) for receiving a succession of a plurality of tubular package units and, separate therefrom, a separation system (8) with a conveying device (9), a separating device (15) and a packaging unit dispenser (16), which can brought into engagement with the storage container (1), **characterised in that** the cover (2) is opened in an automated manner through the engagement between the storage container (1) and the separation system (8), and the foremost package unit (21) is brought into an engagement with the conveying device (9) and conveyed by the latter to the package unit dispenser (16).

2. Device according to claim 1, wherein the storage container (1)
a) comprises, at a dispensing opening, a support surface (7) with a forced guide (6) for the succession of packaging units; and/or
b) comprises an element for position recognition (5), which determines the position between the storage container (1) and the separation system (8) .

3. Device according to claim 1 or 2, wherein the storage container (1) comprises a starting ramp (4), which when the storage container (1) is brought into contact with the separation system (8), come into contact with a lever (10) of the separation system (8), through which a support surface (7) of the storage container is pushed under a conveying device (9) of the separation system (8).

4. Device according to any one of claims 1 to 3, wherein the separation system (8) comprises a conveying element which transports the succession of package unit from a inlet opening in the separation system (8) to the dispensing opening of the separation system (16), wherein the conveying device (9) comprises a spring-loaded lever-belt drive (10) which interacts with a starting ramp (4) in the storage container.

5. Device according to any one of claims 1 to 4, wherein the separation system (8) comprises a reading unit that transmits package unit information to a computer.

6. Device according to any one of claims 1 to 5, wherein the separation system (8) comprises at least one emitter (11) and at least one receiver (12), which determine the positions of the package units within the separation device (8).

7. Device according to any one of claims 1 to 6, wherein the separation system (8)
a) comprises at least one display (13) for displaying;
b) comprises at least one separating device (15) for separating the individual package units (21);
c) comprises an element for position recognition (5); and/or
d) comprises a holding down device (14), which keeps the package units in a determined position.

8. Device according to any one of claims 1 to 7, wherein the cover (2) of the storage container (1) comprises a lock for preventing a manipulation of the package units (21) contained therein, wherein preferably locking takes place by means of a key or similar object, a numerical combination, biometric identification, smartphone or other forms of identification.

9. Device according to any one of claims 2 to 8, wherein the forced guide (6) comprises guiding elements which minimise or prevent lateral movements of the package units (21).

10. Device according to any one of claims 2 to 9, wherein the element for position recognition (5) comprises a contact surface on the storage container (1) and a position sensor on the separation system (8).

11. Method of dispensing a particular package unit to a patient with a device according to any one of claims 1 to 10, wherein the method comprises the following steps:
a) bringing a storage container (1) into contact with a separation system (8), whereby a starting ramp (4) of the storage container (1) pushes a lever-belt drive (10) of the separation system upwards and a support surface (7) of the storage container (1) is pushed under the conveying belt;
b) opening a cover (2) of the storage container (1);
c) determination of an end position between the storage container (1) and the separation system (8) ;
d) lowering of a spring-loaded belt drive onto the support surface (7) and activation of the conveying device (9);
e) transporting the package unit (21) from an inlet opening to a package unit dispenser (16) within the separation system (8);
f) determination of the position of the package unit (21) within the separation system (8);
g) fixing of the packaging unit (21) in a certain position; and
h) separation of the packaging unit (21) from the succession.

12. Method according to claim 11, wherein between transporting of the package unit (21) from an inlet opening to the package unit dispenser (16) within the separation system (8) (step e) and determination of the position of the packaging unit (21) within the separation system (8) (step f), checking of the data on the packaging unit takes place.

13. Method according to claim 11 or 12, wherein a lock of the cover (2) of the storage container (1) for filling with package units (21), is opened by means of a key or similar object, a numerical combination, biometric identification, a smartphone and/or other forms of identification.

14. Method according to any one of claims 11 to 13, wherein checking of the data comprises comparison with internal and/or external databases, preferably during which information about the package content, the patient, the forms of administration and/or other information is shown on a display (13) of the separation system (8) or on an external device.

## Revendications

1. Dispositif de distribution d'une unité d'emballage comprenant un récipient de conservation (1) pourvu d'un couvercle (2) et destiné à recevoir une rangée d'une pluralité d'unités d'emballage tubulaires et un dispositif d'individualisation (8) séparé de celles-ci, comportant un dispositif de transport (9), un dispositif de séparation (15) et un distributeur d'unités d'emballage (16) qui peut être mis en prise avec le récipient de conservation (1), **caractérisé en ce que** le couvercle (2) s'ouvre automatiquement par emboîtement entre le récipient de conservation (1) et un dispositif d'individualisation (8) et qu'une unité d'emballage située le plus en avant (21) est mise en prise avec le dispositif de transport (9) et est convoyée par celui-ci vers le distributeur d'unités d'emballage (16).

2. Dispositif selon la revendication 1, dans lequel le récipient de conservation (1)
a) présente, à une ouverture de distribution, un recouvrement (7) doté d'un guide forcé (6) pour la disposition en rangée des unités d'emballage ; et/ou
b) comprend un élément de détection de position (5) qui définit la position entre le récipient de conservation (1) et le dispositif d'individualisation (8).

3. Dispositif selon la revendication 1 ou 2, dans lequel le récipient de conservation (1) présente une rampe d'arrivée (4) qui, lors de la mise en contact du récipient de conservation (1) avec le dispositif d'individualisation (8), arrive en contact avec un levier (10) du dispositif d'individualisation (8), ce qui a pour effet qu'un recouvrement (7) du récipient de conservation est glissé en-dessous d'un sens de transport (9) du dispositif d'individualisation (8).

4. Dispositif selon une des revendications 1 à 3, dans lequel le dispositif d'individualisation (8) comprend un élément de transport qui transporte les unités d'emballage en rangée depuis une ouverture d'introduction dans le dispositif d'individualisation (8) jusqu'à l'ouverture de distribution du dispositif d'individualisation (16), le dispositif de transport (9) comprenant une propulsion à levier et courroie à palier à ressort (10) qui interagit avec une rampe d'arrivée (4) dans le récipient de conservation.

5. Dispositif selon une des revendications 1 à 4, dans lequel le dispositif d'individualisation (8) comprend un lecteur qui transfère des informations des unités d'emballage à un ordinateur.

6. Dispositif selon une des revendications 1 à 5, dans lequel le dispositif d'individualisation (8) comprend au moins un émetteur (11) et au moins un récepteur (12) qui définissent les positions des unités d'emballage à l'intérieur du dispositif d'individualisation (8).

7. Dispositif selon une des revendications 1 à 6, dans lequel le dispositif d'individualisation (8)
a) comprend au moins un afficheur (13) pour la représentation ;
b) comprend au moins un dispositif de séparation (15) pour séparer les unités d'emballage individuelles (21) ;
c) comprend un élément de détection de position (5) ; et/ou
d) comprend un serre-flan (14) qui maintient les unités d'emballage dans une certaine position.

8. Dispositif selon une des revendications 1 à 7, dans lequel le couvercle (2) du récipient de conservation (1) présente un système de verrouillage pour empêcher une manipulation des unités d'emballage (21) qu'il contient, le verrouillage ayant lieu de préférence au moyen d'une clé ou d'un objet similaire, d'une combinaison de chiffres, d'une identification biométrique, d'un Smartphone et/ou d'autres identifiants.

9. Dispositif selon une des revendications 2 à 8, dans lequel le guide forcé (6) comprend des éléments de guidage qui minimisent ou empêchent le mouvement latéral des unités d'emballage (21).

10. Dispositif selon une des revendications 2 à 9, dans lequel l'élément de détection de position (5) comprend une surface de contact au niveau du récipient de conservation (1) et un interrupteur de position au niveau du dispositif d'individualisation (8).

11. Procédé de distribution d'une certaine unité d'emballage à un patient avec un dispositif selon une des revendications 1 à 10, ce procédé comprenant les étapes suivantes :
a) mise en contact d'un récipient de conservation (1) avec un dispositif d'individualisation (8), ce qui a pour effet qu'une lampe d'arrivée (4) du récipient de conservation (1) pousse vers le haut une propulsion à levier et courroie (10) du dispositif d'individualisation et qu'un recouvrement (7) du récipient de conservation (1) se glisse sous la courroie de transport ;
b) ouverture d'un couvercle (2) du récipient de conservation (1) ;
c) constatation d'une position finale entre le récipient de conservation (1) et le dispositif d'individualisation (8) ;
d) descente d'une propulsion à courroie à palier à ressort sur le recouvrement (7) et activation du dispositif de transport (9) ;
e) transport de l'unité d'emballage (21) depuis une ouverture d'entrée jusqu'à un distributeur d'unités d'emballage (16) dans le récipient de conservation avec un dispositif d'individualisation (8) ;
f) définition de la position de l'unité d'emballage (21) dans le dispositif d'individualisation (8),
g) fixation de l'unité d'emballage (21) à une certaine position ; et
h) séparation d'une unité d'emballage (21) de la rangée.

12. Procédé selon la revendication 11, dans lequel, entre le transport de l'unité d'emballage (21) d'une position d'entrée au distributeur d'unités d'emballage (16) à l'intérieur du dispositif d'individualisation (8) (étape e) et la définition de la position de l'unité d'emballage (21) à l'intérieur du dispositif d'individualisation (8) (étape f), il y a un contrôle des données sur l'unité d'emballage.

13. Procédé selon la revendication 11 ou 12, dans lequel un obturateur du couvercle (2) du récipient de conservation (1) à garnir avec des unités d'emballage (21) est ouvert au moyen d'une clé ou d'un objet similaire, d'une combinaison de chiffres, d'une identification biométrique, d'un Smartphone et/ou d'autres identifiants.

14. Procédé selon une des revendications 11 à 13, dans lequel le contrôle de données comprend le rapprochement avec des banques de données internes et/ou externes, des informations concernant le contenu de l'emballage, le patient, les formes d'administration et/ou d'autres informations étant de préférence restituées sur un afficheur (13) du dispositif d'individualisation (8) ou d'un appareil externe.
